# EUROPÄISCHE PATENTSCHRIFT

(11) **EP 2 480 193 B1**
(45) Veröffentlichungstag und Bekanntmachung des Hinweises auf die Patenterteilung: **19.08.2015**
(21) Anmeldenummer: 10754328.2
(22) Anmeldetag: 15.09.2010
(51) Int. Cl.: A61K 8/37, A61K 8/02, A61K 8/92, A61K 8/86, A61Q 15/00

(54) **WASSERFREIE ANTITRANSPIRANT-NONAEROSOLE MIT VERBESSERTER WIRKSTOFFFREISETZUNG**
NON-AEROSOL WATER-FREE ANTIPERSPIRANT IN WHICH ACTIVE SUBSTANCES ARE MORE READILY RELEASED
ANTI-TRANSPIRANTS NON AÉROSOL SANS EAU À LIBÉRATION AMÉLIORÉE D'AGENTS

(30) Priorität: 22.09.2009 DE 102009029671
(43) Veröffentlichungstag der Anmeldung: 01.08.2012
(73) Patentinhaber: Henkel AG & Co. KGaA, 40589 Düsseldorf (DE)
(72) Erfinder: BANOWSKI, Bernhard, 40597 Düsseldorf (DE); BUSE, Nadine, 40724 Hilden (DE)
(86) Internationale Anmeldenummer: PCT/EP2010/063571
(87) Internationale Veröffentlichungsnummer: WO 2011/036082

(56) Entgegenhaltungen:
- WO-A1-98/17238
- WO-A2-2009/083547
- DE-A1- 19 501 288
- US-A- 4 479 887
- US-A- 5 080 830
- US-A1- 2001 051 138

## Beschreibung

Gegenstand der vorliegenden Erfindung sind wasserfreie schweißhemmende Zusammensetzungen zur persönlichen Körperpflege, konfektioniert als Nonaerosol, Stift, Soft Solid, Creme, Gel, nicht-versprühbare Suspension, nicht-versprühbare Lösung oder auf einem Substrat imprägniert, die eine verbesserte Wirkstofffreisetzung des schweißhemmenden Wirkstoffs ermöglichen. Wasserfreie Antitranspirant-Zusammensetzungen, konfektioniert als Nonaerosol, Stift, Creme, Gel, Suspension oder Lösung, enthalten neben den schweißreduzierenden Wirkstoffen in der Regel mindestens ein kosmetisches Öl als Träger für den teilchenförmigen schweißreduzierenden Wirkstoff. Damit sich der im Öl suspendierte Antitranspirantwirkstoff bei der Lagerung nicht absetzt, enthalten handelsübliche Suspensionen, Gele, Cremes und Stifte ein Suspendier- oder Verdickungsmittel, bei Gelen z. B. hydrophob modifizierte Hectorite, wie sie beispielsweise unter der Handelsbezeichnung Bentone Gel von den Firmen Rheox und Elementis Specialties erhältlich sind, bei Stiften Verdickungsmittel wie Fettalkohole und/oder Wachse.

Bei den handelsüblichen wasserfreien Antitranspirant-Zusammensetzungen ist der in dem wasserfreien, ölhaltigen Träger suspendierte Antitranspirantwirkstoff mit einer Ölschicht bedeckt. Diese Ölschicht verzögert allerdings die Freisetzung des Antitranspirantwirkstoffs in die wirksame wasserlösliche Form.

Im Stand der Technik sind aus US 5118497 und US 5017360 wasserhaltige Antitranspirant-Cremes mit einem Wassergehalt von 43,9 Gew.-% bekannt, die mit Hilfe des Handelsprodukts Elfacos ST-37, einem Alkyl-modifizierten Polyether mit der INCI-Bezeichnung PEG-22/Dodecyl Glycol Copolymer, der einen HLB-Wert von 2,4 aufweist, emulgiert sind.

US 6613338 und insbesondere WO 98/17238 A1 offenbaren Stifte, insbesondere Antitranspirant-Stifte, in Form von festen Wasser-in-Öl-Emulsionen mit einem Wassergehalt von 30 - 85 Gew.-%, die mit Hilfe eines Alkyl-modifizierten Polyethers der allgemeinen Formel ACTIVATOR-(I) mit der INCI-Bezeichnung Methoxy PEG-22/ Dodecyl Glycol Copolymer emulgiert sind. Dieser Stand der Technik gab dem Fachmann keinen Hinweis darauf, dass Alkyl-modifizierte Polyether der allgemeinen Formel ACTIVATOR-(I) die Freisetzung des Antitranspirantwirkstoffs aus einer wasserfreien Zusammensetzung verbessern können.

WO 2009/083547 A2 und WO2009/083807 A2 lehren, dass bestimmte Organosiloxan-Oxyalkylen-Copolymere die Freisetzung des Antitranspirantwirkstoffs aus einer wasserfreien Zusammensetzung verbessern können. Als Nachteil dieser Substanzen hat sich aber herausgestellt, dass sie sich unter bestimmten Umständen, insbesondere wenn der kosmetische Träger wenig oder kein Siliconöl enthält, schlecht einarbeiten und homogen einmischen lassen.

DE 19501288A1 offenbart Wasser-in-Öl-Emulsionen, in denen die vorliegend beanspruchten Alkyl-modifizierten Polyether der allgemeinen Formel ACTIVATOR-(I) einen Teil des W/O-Emulsionssystems darstellen.

US 5080830 beschäftigt sich gemäß der Zusammenfassung ebenfalls mit wässrigen und wasserbasierten Zusammensetzungen, die Methoxy PEG-17/Dodecyl Glycol Copolymer als Teil eines Stabilisator- und Dispergiermittelsystems umfasst und dabei insbesondere zur Formulierung von Haarconditionern und Wäscheweichspülmitteln geeignet ist.

US 2001/051138 A1 offenbart Antitranspirantzusammensetzungen in Form einer Wasser-in-Öl-Emulsion, die unter anderem 65 bis 75 Gew.-% einer wässrigen Lösung eines Antitranspirantwirkstoffs und 0,01 bis 8,5 Gew.-% eines Kupplungsmittels enthalten. Als geeignetes Kupplungsmittel ist unter anderem Methoxy PEG-22/Dodecyl Glycol Copolymer offenbart. Das Kupplungsmittel dient dazu, die Emulsion zu stabilisieren und Transparenz herzustellen.

Überraschend wurde nun gefunden, dass die Freisetzung des schweißhemmenden Wirkstoffs aus einer wasserfreien Antitranspirant-Zusammensetzung verbessert werden kann, wenn mindestens ein Alkyl-modifizierter Polyether der allgemeinen Formel ACTIVATOR-(I) 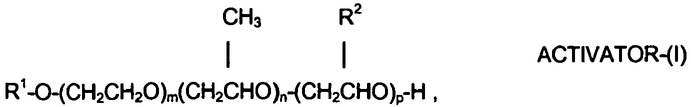 worin
R¹ ein aliphatischer Kohlenwasserstoffrest mit 1 bis 3 C-Atomen,
R² ein aliphatischer Kohlenwasserstoffrest mit 8 bis 30 C-Atomen,
m eine rationale Zahl von 10 bis 50,
n eine rationale Zahl von 0 bis 10,
p eine rationale Zahl von 1 bis 10
bedeutet, enthalten ist.

Gegenstand der vorliegenden Erfindung sind daher schweißhemmende Zusammensetzungen zur persönlichen Körperpflege, konfektioniert als Nonaerosol, Stift, Soft Solid, Creme, Gel, nicht-versprühbare Suspension, nicht-versprühbare Lösung oder auf einem Substrat imprägniert, enthaltend mindestens einen schweißhemmenden Wirkstoff, mindestens ein unter Nörmalbedingungen flüssiges Öl als Träger, 0 - 7 Gew.-%, bevorzugt 0 - 3 Gew.-%, freies Wasser, bezogen auf das Gewicht der Zusammensetzung, und mindestens einen Alkyl-modifizierten Polyether der allgemeinen Formel ACTIVATOR-(I) 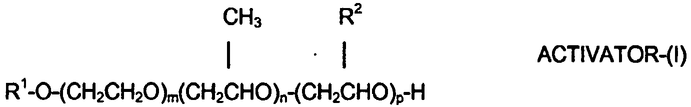 worin
R¹ ein aliphatischer Kohlenwasserstoffrest mit 1 bis 3 C-Atomen,
R² ein aliphatischer Kohlenwasserstoffrest mit 8 bis 30 C-Atomen,
m eine rationale Zahl von 10 bis 50,
n eine rationale Zahl von 0 bis 10,
p eine rationale Zahl von 1 bis 10
bedeutet.

Die erfindungsgemäßen schweißhemmenden Zusammensetzungen auf wasserfreier Basis sind als Stift, Soft Solid, Creme, Gel, Suspension, Lösung oder als mit der Zusammensetzung imprägniertes Substrat (Tuch, Pad, Bausch etc.) konfektioniert.

Alle im Folgenden gemachten Mengenangaben beziehen sich auf das Gewicht der gesamten anwendungsfertigen Zusammensetzung.

"Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Erfindungsgemäß bevorzugte Zusammensetzungen sind dadurch gekennzeichnet, dass der Alkyl-modifizierte Polyether d) ausgewählt ist aus Verbindungen der allgemeinen Formel ACTIVATOR-(I), worin
R¹ ausgewählt ist aus einer Methylgruppe, einer Ethylgruppe, einer n-Propylgruppe und einer 1-Methylethylgruppe, bevorzugt einer Methylgruppe,
R² ausgewählt ist aus einer n-Octyl-, 2-Ethylhexyl-, n-Nonyl-, n-Decyl-, 2-Ethyloctyl-, n-Undecyl-, n-Dodecyl-, 2-Ethyldecyl-, n-Tridecyl-, Myristyl-, n-Pentadecyl-, Cetyl-, Palmityl-, Stearyl-, Elaidyl-, Arachidyl-, Behenyl- oder Cocyl-Gruppe, bevorzugt einer n-Decyl-Gruppe,
m eine rationale Zahl von 12 - 30, bevorzugt 22 - 23, darstellt,
n eine rationale Zahl von 0 - 8, bevorzugt 0 - 4, besonders bevorzugt 0, darstellt,
p eine rationale Zahl von 1 - 9, bevorzugt 4 - 8, besonders bevorzugt 5 - 7, darstellt.

Bevorzugt für die erfindungsgemäße Lehre (Zusammensetzung, Verwendung, Verfahren) sind solche Alkyl-modifizierten Polyether d) der allgemeinen Formel ACTIVATOR-(I), in denen R¹ eine Methylgruppe darstellt

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen R² eine n-Decyl-Gruppe darstellt.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen m eine rationale Zahl von 21 - 23 darstellt.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen m eine rationale Zahl von 22 - 23 darstellt.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen n = 0 ist.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen p eine rationale Zahl von 4 - 5 darstellt.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen p eine rationale Zahl von 6 - 8 darstellt.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen p eine rationale Zahl von 7 - 8 darstellt.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen R¹ eine Methylgruppe, R² eine n-Decyl-Gruppe, m eine rationale Zahl von 22 - 23, n = 0 und p eine rationale Zahl von 4 - 5 darstellt.

Weiterhin bevorzugt für die erfindungsgemäße Lehre (Zusammensetzung, Verwendung, Verfahren) sind solche Alkyl-modifizierten Polyether d) der allgemeinen Formel ACTIVATOR-(I), in denen R¹ eine Methylgruppe, R² eine n-Decyl-Gruppe, m eine rationale Zahl von 22 - 23, n = 0 und p eine rationale Zahl von 7 - 8 darstellt.

Weitere bevorzugte erfindungsgemäße Zusammensetzungen sind dadurch gekennzeichnet, dass der Alkyl-modifizierte Polyether d) der allgemeinen Formel ACTIVATOR-(I), worin R¹ ein aliphatischer Kohlenwasserstoffrest mit 1 bis 3 C-Atomen, R² ein aliphatischer Kohlenwasserstoffrest mit 8 bis 30 C-Atomen, m eine rationale Zahl von 10 bis 50, n eine rationale Zahl von 0 bis 10 und p eine rationale Zahl von 1 bis 10 ist, einen HLB-Wert im Bereich von 5 - 7, bevorzugt 6 - 6,8, besonders bevorzugt 6,2 - 6,5, aufweist.

Weitere bevorzugte erfindungsgemäße Zusammensetzungen sind dadurch gekennzeichnet, dass der Alkyl-modifizierte Polyether d) der allgemeinen Formel ACTIVATOR-(I), worin R¹ ausgewählt ist aus einer Methylgruppe, einer Ethylgruppe, einer n-Propylgruppe und einer 1-Methylethylgruppe, bevorzugt einer Methylgruppe, R² ausgewählt ist aus einer n-Octyl-, 2-Ethylhexyl-, n-Nonyl-, n-Decyl-, 2-Ethyloctyl-, n-Undecyl-, n-Dodecyl-, 2-Ethyldecyl-, n-Tridecyl-, Myristyl-, n-Pentadecyl-, Cetyl-, Palmityl-, Stearyl-, Elaidyl-, Arachidyl-, Behenyl- oder Cocyl-Gruppe, bevorzugt einer n-Decyl-Gruppe, m eine rationale Zahl von 12 - 30, bevorzugt 22 - 23, darstellt, n eine rationale Zahl von 0 - 8, bevorzugt 0 - 4, besonders bevorzugt 0, darstellt, p eine rationale Zahl von 1 - 9, bevorzugt 4 - 8, besonders bevorzugt 5 - 7, darstellt, einen HLB-Wert im Bereich von 5 - 7, bevorzugt 6 - 6,8, besonders bevorzugt 6,2 - 6,5, aufweist

Weitere bevorzugte erfindungsgemäße Zusammensetzungen sind dadurch gekennzeichnet, dass der Alkyl-modifizierte Polyether d) der oben dargestellten allgemeinen Formel ACTIVATOR-(I), worin R¹ ausgewählt ist aus einer Methylgruppe, R² ausgewählt ist aus einer n-Decyl-Gruppe, m eine rationale Zahl von 22 - 23, n = 0 und p eine rationale Zahl von 4 - 8 ist, einen HLB-Wert im Bereich von 5 - 7, bevorzugt 6 - 6,8, besonders bevorzugt 6,2 - 6,5, aufweist.

Weitere bevorzugte erfindungsgemäße Zusammensetzungen sind dadurch gekennzeichnet, dass der Alkyl-modifizierte Polyether d) der oben dargestellten allgemeinen Formel ACTIVATOR-(I) mit R¹ = Methyl, R² = n-Decyl, m = 22, n = 0 und p = 4 - 5 ist und einen HLB-Wert im Bereich von 5 - 7, bevorzugt 6 - 6,8, besonders bevorzugt 6,2 - 6,5, aufweist

Weitere bevorzugte erfindungsgemäße Zusammensetzungen sind dadurch gekennzeichnet, dass der Alkyl-modifizierte Polyether d) der oben dargestellten allgemeinen Formel ACTIVATOR-(I) mit R¹ = Methyl, R² = n-Decyl, m = 22, n = 0 und p = 7 - 8 ist und einen HLB-Wert im Bereich von 5 - 7, bevorzugt 6 - 6,8, besonders bevorzugt 6,2 - 6,5, aufweist.

Erfindungsgemäß bevorzugte Alkyl-modifizierte Polyether der allgemeinen Formel ACTIVATOR-(I) sind erhältlich gemäß dem in US 4479887, Beispiel 1, insbesondere Beispiel 1A und 1D, offenbarten Herstellungsverfahren aus einem C₁-C₃-Alkanolethoxylat und einem 1,2-Epoxy-C₁₀-C₃₂-Alkan. Ein erfindungsgemäß bevorzugter Alkyl-modifizierter Polyether der allgemeinen Formel ACTIVATOR-(I) ist ausgewählt aus Verbindungen mit der INCI-Bezeichnung Methoxy PEG-22/Dodecyl Glycol Copolymer. Eine solche Verbindung ist beispielsweise als Handelsprodukt Elfacos E 200 erhältlich.

Weitere bevorzugte erfindungsgemäße Zusammensetzungen sind dadurch gekennzeichnet, dass mindestens ein Alkyl-modifizierter Polyether d) der oben dargestellten allgemeinen Formel ACTIVATOR-(I) in einer Gesamtmenge von 0,01 - 5 Gew.-%, bevorzugt 0,1 - 3 Gew.%, besonders bevorzugt in einer Gesamtmenge von 0,5 - 2 Gew.-%, außerordentlich bevorzugt in einer Gesamtmenge von 1 - 1,5 Gew.-%, enthalten ist, jeweils bezogen auf das Gesamtgewicht der Zusammensetzung.

Weitere bevorzugte erfindungsgemäße Zusammensetzungen sind dadurch gekennzeichnet, dass Methoxy PEG-22/Dodecyl Glycol Copolymer in einer Gesamtmenge von 0,01 - 5 Gew.-%, bevorzugt 0,1 - 3 Gew.-%, besonders bevorzugt in einer Gesamtmenge von 0,5 - 2 Gew.%, außerordentlich bevorzugt in einer Gesamtmenge von 1 - 1,5 Gew.-%, enthalten ist, jeweils bezogen auf das Gesamtgewicht der Zusammensetzung.

Alkyl-modifizierte Polyether d) der oben dargestellten allgemeinen Formel ACTIVATOR-(I) lassen sich, auch wenn der kosmetische Träger wenig oder kein Siliconöl enthält, gut in die erfindungsgemäßen Zusammensetzungen einarbeiten und homogen einmischen.

Die erfindungsgemäßen Zusammensetzungen sind im Wesentlichen wasserfrei, d. h. sie enthalten 0 bis maximal 7 Gew.-%, bevorzugt 0 bis maximal 3 Gew.-% freies Wasser, außerordentlich bevorzugt 0 bis maximal 2 Gew.-% freies Wasser, jeweils bezogen auf die gesamte Zusammensetzung. Der Gehalt an Kristallwasser, Hydratationswasser oder ähnlich molekular gebundenem Wasser, der in den eingesetzten Bestandteilen, insbesondere in den schweißhemmenden Wirkstoffen enthalten sein kann, stellt im Sinne der vorliegenden Anmeldung kein freies Wasser dar.

Die erfindungsgemäßen Zusammensetzungen enthalten mindestens einen Antitranspirant-Wirkstoff.

Bevorzugte Antitranspirant-Wirkstoffe sind ausgewählt aus den wasserlöslichen adstringierenden anorganischen und organischen Salzen des Aluminiums und Zinks bzw. beliebigen Mischungen dieser Salze.

Alumosilicate und Zeolithe zählen erfindungsgemäß nicht zu den Antitranspirant-Wirkstoffen. Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffs in 95 g Wasser bei 20 °C löslich sind.

Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3H₂O]ₙ, das in nicht-aktivierter oder in aktivierter (depolymerisierter) Form vorliegen kann.

Die Herstellung bevorzugter Antitranspirant-Wirkstoffe ist beispielsweise in US 3887692, US 3904741, US 4359456, GB 2048229 und GB 1347950 offenbart.

Weiterhin bevorzugt sind Aluminiumsesquichlorhydrat, Aluminiumdichlorhydrat, Aluminiumchlorohydrex-Propylenglycol (PG) oder Aluminiumchlorohydrex-Polyethylenglycol (PEG), Aluminium-Glycol-Komplexe, z. B. Aluminium-Propylenglycol-Komplexe, Aluminiumsesquichlorohydrex-PG oder Aluminiumsesquichlorohydrex-PEG, Aluminium-PG-dichlorohydrex oder Aluminium-PEGdichlorohydrex, Aluminiumhydroxid, weiterhin ausgewählt aus Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat und Zinksulfat.

Erfindungsgemäß besonders bevorzugte Antitranspirant-Wirkstoffe sind ausgewählt aus so genannten "aktivierten" Aluminiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in GB 2048229, US 4775528 und US 6010688 offenbart. Aktivierte Aluminiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Aktivierte Aluminiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Aktivierte Aluminiumsalze müssen nicht notwendigerweise als sprühgetrocknetes Pulver eingesetzt werden. Erfindungsgemäß ebenfalls bevorzugte schweißhemmende Wirkstoffe sind nicht-wässrige Lösungen oder Solubilisate eines aktivierten schweißhemmenden Aluminiumsalzes, beispielsweise gemäß US 6010688, die durch den Zusatz einer wirksamen Menge eines mehrwertigen Alkohols, der 3 bis 6 Kohlenstoffatome und 3 bis 6 Hydroxyl-Gruppen, bevorzugt Propylenglycol, Sorbit und Pentaerythrit, aufweist, gegen den Verlust der Aktivierung gegen den raschen Abbau des HPLC-Peak 4:Peak 3-Flächenverhältnisses des Salzes stabilisiert sind. Beispielsweise bevorzugt sind Zusammensetzungen, die in Gewichtsprozent (USP) enthalten: 18 - 45 Gew.-% eines aktivierten Aluminiumsalzes, 55 - 82 Gew.-% mindestens eines wasserfreien mehrwertigen Alkohols mit 3 bis 6 Kohlenstoffatomen und 3 bis 6 Hydroxyl-Gruppen, bevorzugt Propylenglycol, Butylenglycol, Diethylenglycol, Dipropylenglycol, Glycerin, Sorbit und Pentaerythrit, besonders bevorzugt Propylenglycol.

Besonders bevorzugt sind auch Komplexe aktivierter schweißhemmender Aluminiumsalze mit einem mehrwertigen Alkohol, die 20 - 50 Gew.-%, besonders bevorzugt 20 - 42 Gew.-%, aktiviertes schweißhemmendes Aluminiumsalz und 2 - 16 Gew.-% molekular gebundenes Wasser enthalten, wobei der Rest zu 100 Gew.-% mindestens ein mehrwertiger Alkohol mit 3 bis 6 Kohlenstoffatome und 3 bis 6 Hydroxyl-Gruppen ist. Propylenglycol, Propylenglycol/Sorbit-Mischungen und Propylenglycol/Pentaerythrit-Mischungen sind bevorzugte derartige Alkohole. Derartige erfindungsgemäß bevorzugte Komplexe eines aktivierten schweißhemmenden Aluminiumsalzes mit einem mehrwertigen Alkohol sind z. B. offenbart in US 5643558 und US 6245325.

Weitere bevorzugte schweißhemmende Wirkstoffe sind basische Calcium-Aluminiumsalze, wie sie beispielsweise in US 2571030 offenbart sind. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind aktivierte Aluminiumsalze, wie sie beispielsweise in US 6245325 oder US 6042816 offenbart sind, enthaltend 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu Aluminium - Gewichtsverhältnis von 2:1 - 1:20 und bevorzugt 1:1 bis 1:10 bereitzustellen, sowie ein wasserlösliches Calciumsalz in einer solchen Menge, um ein Ca:Al-Gewichtsverhältnis von 1:1 - 1:28 und bevorzugt 1:2 - 1:25 bereitzustellen.

Besonders bevorzugte feste aktivierte schweißhemmende Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:Al-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.%, bevorzugt 4 - 13 Gew.-% molekular gebundenes Wasser (Hydratationswasser), weiterhin soviel wasserlösliches Calciumsalz, dass das Ca:Al-Gewichtsverhältnis 1:1 - 1:28, bevorzugt 1:2 - 1:25, beträgt und soviel Glycin, dass das Glycin zu Al - Gewichtsverhältnis 2:1 - 1:20, bevorzugt 1:1 -1:10, beträgt. Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Calciumsalz, dass das Ca:Al-Gewichtsverhältnis 1:1 - 1:28, bevorzugt 1:2 - 1:25, beträgt und soviel Hydroxyalkansäure, dass das Hydroxyalkansäure zu Al - Gewichtsverhältnis 2:1 - 1:20, bevorzugt 1:1 -1:10, beträgt.

Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind aktivierte Aluminiumsalze, wie sie beispielsweise in US 6902723 offenbart sind, enthaltend 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu Al - Gewichtsverhältnis von 2:1 - 1:20 und bevorzugt 1:1 bis 1:10 bereitzustellen, sowie ein wasserlösliches Strontiumsalz in einer solchen Menge, um ein Sr:Al-Gewichtsverhältnis von 1:1 - 1:28 und bevorzugt 1:2 - 1:25 bereitzustellen.

Besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Aminosäure, dass das Aminosäure zu Al - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:AI-Gewichtsverhältnis 1:1 - 1:28, bevorzugt 1:2 - 1:25, beträgt und soviel Glycin, dass das Glycin zu Al - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:AI-Gewichtsverhältnis 1:1 - 1:28, bevorzugt 1:2 - 1:25, beträgt und soviel Hydroxyalkansäure, dass das Hydroxyalkansäure zu Al - Gewichtsverhältnis 2:1 - 1:20, bevorzugt 1:1 - 1:10, beträgt.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von Al:X 0,9:1 bis 2,1:1 beträgt, wie sie beispielsweise in US 6074632 offenbart sind. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.

Weitere bevorzugte schweißhemmende Wirkstoffe sind in US 6663854 und US 20040009133 offenbart.

Die schweißhemmenden Wirkstoffe können sowohl in solubilisierter als auch in ungelöster, suspendierter Form vorliegen.

Sofern die schweißhemmenden Wirkstoffe in einem mit Wasser nicht mischbaren Träger suspendiert vorliegen, ist es aus Gründen der Produktstabilität bevorzugt, dass die Wirkstoffpartikel eine zahlenmittlere Partikelgröße von 0,1 - 200 µm, bevorzugt 1 - 50 µm, besonders bevorzugt 3 - 20 µm und außerordentlich bevorzugt 5 - 10 µm, aufweisen. Bevorzugte Wirkstoffpartikel weisen eine volumenmittlere Partikelgröße von 0,2 - 220 µm, bevorzugt 3 - 60 µm, besonders bevorzugt 4 - 25 µm, weiterhin bevorzugt 5 - 20 µm und außerordentlich bevorzugt 10 -15,5 µm, auf. Bevorzugte Aluminiumsalze weisen ein molares Metall-zu-Chlorid-Verhältnis von 1,9 -2,1, bzw. für Sesquichlorohydrate von 1,5:1-1,8:1 auf.

Die Antitranspirant-Wirkstoffe können als nicht-wässrige Lösungen oder als glycolische Solubilisate eingesetzt werden.

Besonders bevorzugte erfindungsgemäße Zusammensetzungen sind dadurch gekennzeichnet, dass der mindestens eine Antitranspirant-Wirkstoff in einer Menge von 5 - 40 Gew.%, bevorzugt 10 - 35 Gew.-%, besonders bevorzugt 15 - 28 Gew.% und außerordentlich bevorzugt 23-27 Gew.-%, enthalten ist, bezogen auf das Gesamtgewicht der kristallwasserfreien Aktivsubstanz (USP) in der Gesamtzusammensetzung.

In einer besonders bevorzugten Ausführungsform enthält die Zusammensetzung ein adstringierendes Aluminiumsalz, insbesondere Aluminiumchlorohydrat, besonders bevorzugt Aluminiumchlorohydrat mit einer kristallwasserfreien Aktivsubstanz (USP) von 72 - 88 Gew.%, bezogen auf den Rohstoff tel quel. Bevorzugte nicht-aktivierte Aluminiumchlorohydrate sind beispielsweise pulverförmig als Micro Dry^{®}, Micro Dry^{®} Ultrafine oder Micro Dry^{®}-323 von Summit/Reheis, als Chlorhydrol^{®} (Pulver) sowie in aktivierter Form als Reach^{®} 101, Reach^{®} 103, Reach^{®} 501 von Reheis/Summit oder AACH-7171 von Summit vertrieben wird. Unter der Bezeichnung Reach^{®} 301 wird ein Aluminiumsesquichlorohydrat von Reheis angeboten, das ebenfalls besonders bevorzugt ist.

In einer bevorzugten erfindungsgemäßen Ausführungsform sind die schweißhemmenden Wirkstoffe und gegebenenfalls weitere im Träger unlösliche Wirkstoffe in mindestens einem unter Normalbedingungen flüssigen Öl suspendiert. Zur besseren Anwendbarkeit wird dieser Suspension noch mindestens ein bevorzugt lipophiles Verdickungsmittel als Suspendierhilfe zugesetzt. Weitere bevorzugte erfindungsgemäße Zusammensetzungen sind daher dadurch gekennzeichnet, dass sie mindestens ein lipophiles Verdickungsmittel enthalten.

Bevorzugte erfindungsgemäße Zusammensetzungen sind dadurch gekennzeichnet, dass das mindestens eine lipophile Verdickungsmittel ausgewählt ist aus hydrophobierten Tonmineralien, pyrogenen Kieselsäuren, Bentone-Gelen, Ethylene/Propylene/Styrene Copolymeren, Butylene/ Ethylene/Styrene Copolymeren, Dextrinestern, Siliconelastomeren, unter Normalbedingungen festen Wachsen und/oder Glycerintriestern. Hierunter sind hydrophobierte Tonmineralien besonders bevorzugt. Die erfindungsgemäßen Zusammensetzungen enthalten in einer bevorzugten Ausführungsform mindestens ein Suspensions- oder Verdickungsmittel. Besonders geeignete Verdickungsmittel sind hydrophobierte Tonmineralien wie Montmorillonite, Hectorite und Bentonite, insbesondere Disteardimonium Hectorite und Quatemium-18 Hectorite. Die handelsüblichen Verdickungsmittel stellen diese hydrophobierten Tonmineralien als Pulver oder in Form eines vorgefertigten Gels in Cyclomethicone und gewünschtenfalls eines Gelaktivators, wie z. B. Propylencarbonat, Ethanol oder Wasser, bereit. Weitere geeignete Verdickungsmittel sind pyrogene Kieselsäuren, z. B. die Handelsprodukte der Aerosil^{®}-Serie von Degussa.

Bevorzugte hydrophobierte Tonmineralien sind ausgewählt aus hydrophobierten Montmorilloniten, hydrophobierten Hectoriten und hydrophobierten Bentoniten, besonders bevorzugt aus Disteardimonium Hectorite, Stearalkonium Hectorite, Quaternium-18 Hectorite und Quatemium-18 Bentonite. Die handelsüblichen Verdickungsmittel stellen diese hydrophobierten Tonmineralien als Pulver oder in Form eines Gels in einer Ölkomponente, bevorzugt in Cyclomethicone und/oder einer Nichtsilicon-Ölkomponente, wie z. B. Propylencarbonat, bereit. Die Gelbildung erfolgt durch den Zusatz geringer Mengen an Aktivatoren, wie insbesondere Ethanol oder Propylencarbonat, aber auch Wasser. Derartige Gele sind beispielsweise unter der Handelsbezeichnung Bentone^{®} oder Thixogel erhältlich.

Erfindungsgemäß bevorzugte Zusammensetzungen sind dadurch gekennzeichnet, dass sie mindestens ein hydrophobiertes Tonmineral in einer Gesamtmenge von 0,5 - 10 Gew.-%, bevorzugt 1 - 7 Gew.%, besonders bevorzugt 2 - 6 Gew.%, außerordentlich bevorzugt 3 - 5 Gew.-%, jeweils bezogen auf das Gesamtgewicht der erfindungsgemäßen Zusammensetzung, enthalten. Derartige hydrophobierte Tonmineralien benötigen üblicherweise als Aktivator Ethanol oder Propylencarbonat in einer Menge von 0,3 - 3 Gew.%, bevorzugt 0,5 - 2 Gew.%, jeweils bezogen auf das Gesamtgewicht der erfindungsgemäßen Zusammensetzung.

Weitere erfindungsgemäß bevorzugte lipophile Verdickungsmittel sind ausgewählt aus pyrogenen Kieselsäuren (Silica), z. B. den Handelsprodukten der Aerosil^{®}-Serie von Evonik Degussa. Besonders bevorzugt sind hydrophobierte pyrogene Kieselsäuren, besonders bevorzugt Silica Silylate und Silica Dimethyl Silylate.

Erfindungsgemäß bevorzugte Zusammensetzungen sind dadurch gekennzeichnet, dass sie mindestens eine pyrogene Kieselsäure, bevorzugt mindestens eine hydrophobierte pyrogene Kieselsäure, in einer Gesamtmenge von 0,5 - 10 Gew.%, bevorzugt 0,8 - 5 Gew.%, besonders bevorzugt 1 - 4 Gew.-%, außerordentlich bevorzugt 1,5 - 2 Gew.%, jeweils bezogen auf das Gesamtgewicht der erfindungsgemäßen Zusammensetzung, enthalten.

Weitere erfindungsgemäß bevorzugte Zusammensetzungen sind dadurch gekennzeichnet, dass sie mindestens eine hydrophile pyrogene Kieselsäure, bevorzugt in einer Gesamtmenge von 0,1 - 10 Gew.-%, besonders bevorzugt 0,3 - 5 Gew.-%, bevorzugt 1 - 2 Gew.-%, außerordentlich bevorzugt 0,7 - 1 Gew.-%, jeweils bezogen auf das Gesamtgewicht der erfindungsgemäßen Zusammensetzung, enthalten. Bevorzugte hydrophile pyrogene Kieselsäuren sind unter den Handelsbezeichnungen Aerosil 200 (INCI: Silica) und Aerosil 300 (INCI: Silica) erhältlich. Weitere erfindungsgemäß bevorzugte Zusammensetzungen sind dadurch gekennzeichnet, dass sie mindestens eine hydrophobierte pyrogene Kieselsäure und mindestens eine hydrophile Kieselsäure enthalten.

Weitere erfindungsgemäß bevorzugte lipophile Verdickungsmittel sind ausgewählt aus Ethylene/ Propylene/Styrene Copolymeren und Butylene/Ethylene/Styrene Copolymeren. Besonders bevorzugt werden die Copolymere als vorverdicktes Öl-basiertes Gel eingesetzt.

Erfindungsgemäß bevorzugte Zusammensetzungen sind dadurch gekennzeichnet, dass sie mindestens ein Ethylene/Propylene/Styrene Copolymer und/oder Butylene/Ethylene/Styrene Copolymer in einer Gesamtmenge von 0,05 - 3 Gew.-%, bevorzugt 0,1 - 2 Gew.-%, besonders bevorzugt 0,2 - 1,0 Gew.-%, außerordentlich bevorzugt 0,3 - 0,5 Gew.-%, jeweils bezogen auf das Gesamtgewicht der erfindungsgemäßen Zusammensetzung, enthalten.

Weitere erfindungsgemäß bevorzugte lipophile Verdickungsmittel sind ausgewählt aus Siliconelastomeren. Eine weitere bevorzugte Ausführungsform der Erfindung ist dadurch gekennzeichnet, dass mindestens ein Siliconelastomer, das erhältlich ist durch die Vernetzung eines Organopolysiloxans, das mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist, enthalten ist.

Erfindungsgemäß besonders bevorzugte Organopolysiloxane mit mindestens 2 C₂ - C₁₀ - Alkenyl-Gruppen mit terminaler Doppelbindung im Molekül sind ausgewählt aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen.

Erfindungsgemäß besonders bevorzugte vernetzende Organopolysiloxane mit mindestens zwei Silicon-gebundenen Wasserstoffatomen sind ausgewählt aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogen-siloxan-Copolymeren. Erfindungsgemäß besonders bevorzugte Siliconelastomere, die als Rohstoff bereits in einem bei Raumtemperatur unter Normalbedingungen flüssigen Silicon vorgequollen vorliegen und ein Silicon-basiertes Gel darstellen, sind kommerziell erhältlich, beispielsweise unter den Handelsnamen SFE 168, ein Cyclomethicone (and) Dimethicone/Vinyl Dimethicone Crosspolymer von GE Silicones, Vinyl Dimethicone Crosspolymere, enthalten in KSG-15 (Cyclomethicone (and) Dimethicone/Vinyl Dimethicone Crosspolymer, Siliconelastomergehalt 4 -10 Gew.-%), KSG-16 (Dimethicone (and) Dimethicone/Vinyl Dimethicone Crosspolymer, Siliconelastomergehalt 20 - 30 Gew.-%), KSG-17 (Cyclomethicone (and) Dimethicone/Vinyl Dimethicone Crosspolymer), KSG-18 (Phenyl Trimethicone (and) Dimethicone/Phenyl Vinyl Dimethicone Crosspolymer, Siliconelastomergehalt 10 - 20 Gew.-%); and KSG-20, erhältlich von Shin Etsu Silicones of America (Akron, Ohio) und von Grant Industries Inc. (Elmwood Park, NJ) die Produkte aus der Gransil^{®}-Serie. Eine weitere bevorzugte Ausführungsform der Erfindung ist dadurch gekennzeichnet, dass das Siliconelastomer durch die Vernetzung eines Organopolysiloxans, das mindestens 2 C₂ - C₁₀-Alkenylgruppen mit terminaler Doppelbindung in jedem Molekül enthält, mit mindestens einem alpha, omega-Dien erhältlich ist. Erfindungsgemäß besonders bevorzugte vernetzende alpha, omega-Diene weisen die Formel CH₂=CH(CH₂)ₓCH=CH₂ mit x = 1 - 20 auf. Besonders bevorzugte alpha, omega-Diene sind ausgewählt aus 1,4-Pentadien, 1,5-Hexadien, 1,6-Heptadien, 1,7-Octadien, 1,8-Nonadien, 1,11-Dodecadien, 1,13-Tetradecadien und 1,19- Eicosadien. Erfindungsgemäß bevorzugte Zusammensetzungen sind dadurch gekennzeichnet, dass sie mindestens ein Siliconelastomer in einer Gesamtmenge von 0,05 - 3 Gew.-%, bevorzugt 0,1 - 2 Gew.-%, besonders bevorzugt 0,15 - 0,5 Gew.-%, außerordentlich bevorzugt 0,2 - 0,3 Gew.-%, jeweils bezogen auf das Gesamtgewicht der erfindungsgemäßen Zusammensetzung, enthalten. Weitere bevorzugte erfindungsgemäße Zusammensetzungen sind dadurch gekennzeichnet, dass sie mindestens ein Silicongummi enthalten. Überraschend wurde festgestellt, dass durch den Zusatz eines Silicongummis die schweißhemmende und deodorierende Wirkung der erfindungsgemäßen Zusammensetzungen weiter verlängert werden kann. Ohne an diese Theorie gebunden sein zu wollen, wird vermutet, dass das Silicongummi auf der Haut einen Film bildet, durch den die schweißhemmenden Wirkstoffpartikel besser auf der Haut haften.

Ein erfindungsgemäß besonders bevorzugtes Silicongummi ist ausgewählt aus Siliconpolymeren mit der INCI-Bezeichnung Dimethiconol. Bevorzugt werden diese Dimethiconole in einer niedrigkonzentrierten Lösung in Cyclomethicone oder Dimethicone mit einer kinematischen Viskosität von 0,65 cSt bis maximal 10 cSt eingesetzt. Besonders bevorzugte Dimethiconole sind von Dow Corning unter den Handelsnamen Dow Corning 1401, Dow Corning 1403 und Dow Corning 1501 erhältlich; diese Produkte enthalten 10 bis 13 Gew.-% Dimethiconol in Cyclomethicone oder Dimethicone.

Erfindungsgemäß bevorzugte Zusammensetzungen sind dadurch gekennzeichnet, dass sie mindestens ein Silicongummi in einer Gesamtmenge von 0,01 - 1,0 Gew.-%, bevorzugt 0,05 - 0,2 Gew.-%, besonders bevorzugt 0,1 - 0,15 Gew.-%, jeweils bezogen auf das Gesamtgewicht der erfindungsgemäßen Zusammensetzung, enthalten.

Die erfindungsgemäßen Zusammensetzungen enthalten mindestens ein Öl als Trägerfluid. Erfindungsgemäß bevorzugte Antitranspirant-Zusammensetzungen enthalten 30 - 95 Gew.-%, bevorzugt 40 - 93 Gew.-%, besonders bevorzugt 50 - 90 Gew.-%, außerordentlich bevorzugt 55 - 85 Gew.-%, jeweils bezogen auf die gesamte Zusammensetzung, an mindestens einem unter Normalbedingungen flüssigen kosmetischen Öl. Weitere erfindungsgemäß bevorzugte Antitranspirant-Zusammensetzungen enthalten insgesamt 35- 60 Gew.-%, bevorzugt 40 - 55 Gew.-%, besonders bevorzugt 45 - 50 Gew.-%, jeweils bezogen auf die gesamte Zusammensetzung, an mindestens einem unter Normalbedingungen flüssigen kosmetischen Öl. Auch eine Gesamtmenge an unter Normalbedingungen flüssigen kosmetischen Ölen von 42, 43, 44, 46, 47, 48, 49, 51, 52, 53, 55, 58, 60, 63, 65, 68, 70, 73, 75, 78 oder 80 Gew.-%, jeweils bezogen auf die gesamte Zusammensetzung, kann erfindungsgemäß besonders bevorzugt sein, wobei eine Gesamtmenge von 45 - 55 Gew.-% besonders bevorzugt ist.

Bei den kosmetischen Ölen unterscheidet man flüchtige und nicht-flüchtige Öle. Unter nichtflüchtigen Ölen versteht man solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man solche Öle, die bei 20 °C und einem Umgebungsdruck von 1013 hPa einen Dampfdruck von 2,66 Pa - 40000 Pa (0,02 mm - 300 mm Hg), bevorzugt 12 - 12000 Pa (0,1 - 90 mm Hg), besonders bevorzugt 13 - 8000 Pa, außerordentlich bevorzugt 30 - 3000 Pa, weiterhin bevorzugt 100 - 400 Pa, aufweisen.

Erfindungsgemäß bevorzugte kosmetische Öle sind ausgewählt aus flüchtigen Siliconölen, zu denen z. B. Dialkyl- und Alkylarylsiloxane, wie beispielsweise Cyclotetrasiloxan, Cyclopentasiloxan, Cyclohexasiloxan, Dimethylpolysiloxan, niedermolekulares Phenyl Trimethicone und Methylphenylpolysiloxan, aber auch Hexamethyldisiloxan, Octamethyltrisiloxan und Decamethyltetrasiloxan zählen. Besonders bevorzugt sind flüchtige Siliconöle, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning (Dampfdruck bei 20°C ca. 13 - 15 Pa) enthalten sind. Ebenfalls besonders bevorzugt sind flüchtige lineare Siliconöle mit 2 - 10 Siloxaneinheiten, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/oder L₄, bevorzugt solche Mischungen, wie sie z. B. in den Handelsprodukten DC 2-1184, Dow Corning^{®} 200 (0,65 cSt) und Dow Corning^{®} 200 (1,5 cSt) von Dow Corning enthalten sind. Ein weiteres bevorzugtes flüchtiges Siliconöl ist ein niedermolekulares Phenyl Trimethicone mit einem Dampfdruck bei 20°C von etwa 2000 Pa, wie es z. B. von GE Bayer Silicones/Momentive unter der Bezeichnung Baysilone Fluid PD 5 erhältlich ist.

Flüchtige Siliconöle sind hervorragend geeignete Trägeröle für erfindungsgemäß bevorzugte Antitranspirant-Zusammensetzungen, da sie ihnen ein angenehmes Hautgefühl und eine geringe Kleideranschmutzung verleihen. Erfindungsgemäß besonders bevorzugte Antitranspirant-Zusammensetzungen sind daher durch einen Gehalt an mindestens einem flüchtigen Siliconöl von 10 - 95 Gew.-%, bevorzugt 30 - 80 Gew.-%, besonders bevorzugt 40 - 70 Gew.-%, außerordentlich bevorzugt 50 - 60 Gew.-%, jeweils bezogen auf die gesamte Zusammensetzung, gekennzeichnet.

Neben oder an Stelle des mindestens einen flüchtigen Siliconöls kann auch mindestens ein flüchtiges Nichtsiliconöl enthalten sein. Bevorzugte flüchtige Nichtsiliconöle sind ausgewählt aus C₈-C₁₆-Isoparaffinen, insbesondere aus Isodecan, Isoundecan, Isododecan, Isotridecan, Isotetradecan, Isopentadecan, und Isohexadecan, sowie Mischungen hiervon. Bevorzugt sind C₁₀-C₁₃-Isoparaffin-Mischungen, insbesondere solche mit einem Dampfdruck bei 20°C von etwa 10 - 400 Pa, bevorzugt 13 - 300 Pa. Dieses mindestens eine flüchtige Nichtsiliconöl ist bevorzugt in einer Gesamtmenge von 10 - 95 Gew.-%, bevorzugt 20 - 70 Gew.-%, besonders bevorzugt 25 - 50 Gew.%, außerordentlich bevorzugt 30 - 40 Gew.-%, jeweils bezogen auf die gesamte Zusammensetzung, enthalten.

Aufgrund des trockeneren Hautgefühls und der schnelleren Wirkstofffreisetzung sind als Trägeröl flüchtige Siliconöle, Isoparaffine, insbesondere ausgewählt aus Isodecan, Isoundecan, Isododecan, Isotridecan, Isotetradecan, Isopentadecan, Isohexadecan oder Isoeicosan, sowie Mischungen von flüchtigen Siliconölen und Isoparaffinen, insbesondere ausgewählt aus Isododecan, Isohexadecan und Isoeicosan, besonders bevorzugt.

Erfindungsgemäß bevorzugte Zusammensetzungen sind dadurch gekennzeichnet, dass das mindestens eine unter Normalbedingungen flüssige Träger-Öl b) mindestens ein Isoparaffinöl, insbesondere ausgewählt aus Isodecan, Isoundecan, Isododecan, Isotridecan, Isotetradecan, Isopentadecan, Isohexadecan und Isoeicosan, umfasst.

Weitere erfindungsgemäß bevorzugte Zusammensetzungen sind dadurch gekennzeichnet, dass das unter Normalbedingungen flüssige Träger-Öl b) eine Mischung aus b)i) einem flüchtigen Siliconöl, ausgewählt aus Cyclomethicone und linearen Polydimethylsiloxanen mit 2 - 10 Siloxaneinheiten, und b)ii) mindestens einem Isoparaffinöl, ausgewählt aus Isodecan, Isoundecan, Isododecan, Isotridecan, Isotetradecan, Isopentadecan, Isohexadecan und Isoeicosan, umfasst.

Neben den vorgenannten, üblicherweise als "flüchtigen" Siliconölen bezeichneten Substanzen sowie neben den vorgenannten flüchtigen Nichtsiliconölen können erfindungsgemäß besonders bevorzugte Antitranspirant-Zusammensetzungen weiterhin mindestens ein nichtflüchtiges kosmetisches Öl, ausgewählt aus nichtflüchtigen Siliconölen und nichtflüchtigen Nichtsiliconölen, enthalten. Das mindestens eine nichtflüchtige Öl gleicht den negativen Effekt des flüchtigen Öls auf das Rückstandsverhalten erfindungsgemäß bevorzugter Antitranspirant-Zusammensetzungen aus. Durch die relativ schnelle Verdunstung der flüchtigen Öle können feste, unlösliche Bestandteile, insbesondere die Antitranspirantwirkstoffe, auf der Haut als unschöner Rückstand sichtbar werden. Diese Rückstände können erfolgreich mit einem nichtflüchtigen Öl maskiert werden. Außerdem können mit einem Gemisch aus nichtflüchtigem und flüchtigem Öl Parameter wie Hautgefühl, Sichtbarkeit des Rückstands und Stabilität der Suspension feinreguliert und besser an die Bedürfnisse der Verbraucher angepasst werden.

Bevorzugte nichtflüchtige Siliconöle sind ausgewählt aus höhermolekularen linearen Dimethylpolysiloxanen, im Handel erhältlich z. B. unter der Bezeichnung Dow Corning^{®} 190, Dow Corning^{®} 200 Fluid mit kinematischen Viskositäten (25°C) im Bereich von 5 - 100 cSt, bevorzugt 6 - 50 cSt oder auch 5 - 10 cSt, und Baysilon^{®} 350 M (mit einer kinematischen Viskosität (25°C) von etwa 350 cSt).

Erfindungsgemäß ebenfalls bevorzugte Siliconöle sind ausgewählt aus Siliconen der Formel (Sil-1), wobei x ausgewählt ist aus ganzen Zahlen von 1 - 20.

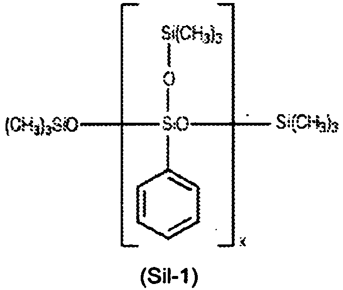

Ein bevorzugtes Siliconöl der Formel (Sil-1) ist unter der INCI-Bezeichnung Phenyl Trimethicone in verschiedenen Qualitäten, Viskositäten und Flüchtigkeiten erhältlich. Ein nicht-flüchtiges Phenyl Trimethicone ist beispielsweise von Dow Corning unter der Bezeichnung Dow Corning 556 erhältlich.

Erfindungsgemäß besonders bevorzugte nichtflüchtige Nichtsiliconöle sind ausgewählt aus den Estern der linearen oder verzweigten gesättigten oder nichtflüchtige Nichtsiliconöle ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Hierunter sind Isopropylpalmitat, Isopropylstearat, Isopropylmyristat, 2-Ethylhexylpalmitat (Cegesoft^{®} C 24) und 2-Ethylhexylstearat (Cetiol^{®} 868) außerordentlich bevorzugt. Ebenfalls bevorzugt sind Hexyldecylstearat (Eutanol^{®} G16S), Hexyldecyllaurat, Isononylisononanoat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Hexyllaurat, n-Decyloleat, Oleyloleat, Oleylerucat, Erucyloleat und Erucylerucat.

Weitere erfindungsgemäß bevorzugte nichtflüchtige Nichtsiliconöle sind ausgewählt aus den Benzoesäureestern von linearen oder verzweigten C₈₋₂₂-Alkanolen. Besonders bevorzugt sind Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD, wobei Benzoesäure-C12-C15-alkylester außerordentlich bevorzugt sind. Ein weiteres erfindungsgemäß bevorzugtes nichtflüchtiges Nichtsiliconöl ist Triethylcitrat. Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether (Witconol^{®} APM).

Weitere erfindungsgemäß besonders bevorzugte nichtflüchtige Nichtsiliconöle sind ausgewählt aus den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether (Ucon Fluid^{®} AP), PPG-9-Butylether (Breox^{®} B25), PPG-10-Butandiol (Macol^{®} 57), PPG-15-Stearylether (Arlamol^{®} E) und Glycereth-7-diisononanoat.

Natürliche und synthetische Kohlenwasserstoffe, wie beispielsweise Paraffinöle, C₁₈-C₃₀-Isoparaffine, insbesondere Isoeicosan, Polyisobutene oder Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan (erhältlich z. B. unter dem Handelsnamen Cetiol^{®}S von Cognis), gehören ebenfalls zu den erfindungsgemäß bevorzugten nichtflüchtigen Nichtsiliconölen.

Weitere erfindungsgemäß bevorzugte nichtflüchtige Nichtsiliconöle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanol^{®} G 16), Octyldodecanol (Eutanol^{®} G) und 2-Ethylhexylalkohol.

Weitere bevorzugte nichtflüchtige Nichtsiliconöle sind ausgewählt aus Mischungen aus Guerbetalkoholen und Guerbetalkoholestern, z.B. dem Handelsprodukt Cetiol^{®} PGL (Hexyldecanol und Hexyldecyllaurat).

Weitere erfindungsgemäß bevorzugte nichtflüchtige Nichtsiliconöle sind ausgewählt aus den Einfach- und Mehrfachestem von Milchsäure, Citronensäure, Weinsäure oder Adipinsäure mit einem zwei-, drei- oder vierwertigen Alkohol mit 2 bis 9 Kohlenstoffatomen. Besonders bevorzugte Ester dieser Art sind ausgewählt aus Ethylenglycolmonolactat, Ethylenglycolmonocitrat, Ethylenglycolmonotartrat, Ethylenglycolmonoadipat, Ethylenglycoldilactat, Ethylenglycoldicitrat, Ethylenglycolditartrat, Ethylenglycoldiadipat, 1,2-Propylenglycolmonolactat, 1,2-Propylenglycolmonocitrat, 1,2-Propylenglycolmonotartrat, 1,2-Propylenglycolmonoadipat, 1,2-Propylenglycoldilactat, 1,2-Propylenglycoldicitrat, 1,2-Propylenglycolditartrat, 1,2-Propylenglycoldiadipat, 1,3-Propylenglycolmonolactat, 1,3-Propylenglycolmonocitrat, 1,3-Propylenglycolmonotartrat, 1,3-Propylenglycolmonoadipat, 1,3-Propylenglycoldilactat, 1,3-Propylenglycoldicitrat, 1,3-Propylenglycolditartrat, 1,3-Propylenglycoldiadipat, 1,2-Butylenglycolmonolactat, 1,2-Butylenglycolmonocitrat, 1,2-Butylenglycolmonotartrat, 1,2-Butylenglycolmonoadipat, 1,2-Butylenglycoldilactat, 1,2-Butylenglycoldicitrat, 1,2-Butylenglycolditartrat, 1,2-Butylenglycoldiadipat, 1,3-Butylenglycolmonolactat, 1,3-Butylenglycolmonocitrat, 1,3-Butylenglycolmonotartrat, 1,3-Butylenglycolmonoadipat, 1,3-Butylenglycoldilactat, 1,3-Butylenglycoldicitrat, 1,3-Butylenglycolditartrat, 1,3-Butylenglycoldiadipat, 1,4-Butylenglycolmonolacat, 1,4-Butylenglycolmonocitrat, 1,4-Butylenglycolmonotartrat, 1,4-Butylenglycolmonoadipat, 1,4-Butylenglycoldilactat, 1,4-Butylenglycoldicitrat, 1,4-Butylenglycolditartrat, 1,4-Butylenglycoldiadipat, 1,2-Pentandiolmonolactat, 1,2-Pentandiolmonocitrat, 1,2-Pentandiolmonotartrat, 1,2-Pentandiolmonoadipat, 1,2-Pentandioldilactat, 1,2-Pentandioldicitrat, 1,2-Pentandiolditartrat, 1,2-Pentandioldiadipat, 1,3-Pentandiolmonolactat, 1,3-Pentandiolmonocitrat, 1,3-Pentandiolmonotartrat, 1,3-Pentandiolmonoadipat, 1,3-Pentandioldilactat, 1,3-Pentandioldicitrat, 1,3-Pentandiolditartrat, 1,3-Pentandioldiadipat, 1,4-Pentandiolmonolactat, 1,4-Pentandiolmonocitrat, 1,4-Pentandiolmonotartrat, 1,4-Pentandiolmonoadipat, 1,4-Pentandioldilactat, 1,4-Pentandioldicitrat, 1,4-Pentandiolditartrat, 1,4-Pentandioldiadipat, 1,5-Pentandiolmonolactat, 1,5-Pentandiolmonocitrat, 1,5-Pentandiolmonotartrat, 1,5-Pentandiolmonoadipat, 1,5-Pentandioldilactat, 1,5-Pentandioldicitrat, 1,5-Pentandiolditartrat, 1,5-Pentandioldiadipat, 1,2-Hexandiolmonolactat, 1,2-Hexandiolmonocitrat, 1,2-Hexandiolmonotartrat, 1,2-Hexandiolmonoadipat, 1,2-Hexandioldilactat, 1,2-Hexandioldicitrat, 1,2-Hexandiolditartrat, 1,2-Hexandioldiadipat, 1,3-Hexandiolmonolactat, 1,3-Hexandiolmonocitrat, 1,3-Hexandiolmonotartrat, 1,3-Hexandiolmonoadipat, 1,3-Hexandioldilactat, 1,3-Hexandioldicitrat, 1,3-Hexandiolditartrat, 1,3-Hexandioldiadipat, 1,4-Hexandiolmonolactat, 1,4-Hexandiolmonocitrat, 1,4-Hexandiolmonotartrat, 1,4-Hexandiolmonoadipat, 1,4-Hexandioldilactat, 1,4-Hexandioldicitrat, 1,4-Hexandiolditartrat, 1,4-Hexandioldiadipat, 1,5-Hexandiolmonolactat, 1,5-Hexandiolmonocitrat, 1,5-Hexandiolmonotartrat, 1,5-Hexandiolmonoadipat, 1,5-Hexandioldiladat, 1,5-Hexandioldicitrat, 1,5-Hexandiolditartrat, 1,5-Hexandioldiadipat, 1,6-Hexandiolmonolactat, 1,6-Hexandiolmonocitrat, 1,6-Hexandiolmonotartrat, 1,6-Hexandiolmonoadipat, 1,6-Hexandioldilactat, 1,6-Hexandioldicitrat, 1,6-Hexandiolditartrat, 1,6-Hexandioldiadipat, 2-Ethylhexan-1,2-diolmonolactat, 2-Ethylhexan-1,2-diolmonocitrat, 2-Ethylhexan-1,2-diolmonotartrat, 2-Ethylhexan-1,2-diolmonoadipat, 2-Ethylhexan-1,2-dioldilactat, 2-Ethylhexan-1,2-dioldicitrat, 2-Ethylhexan-1,2-diolditartrat, 2-Ethylhexan-1,2-dioldiadipat, 2-Ethylhexan-1,3-diolmonolactat, 2-Ethylhexan-1,3-diolmonocitrat, 2-Ethylhexan-1,3-diolmonotartrat, 2-Ethylhexan-1,3-diolmonoadipat, 2-Ethylhexan-1,3-dioldilactat, 2-Ethylhexan-1,3-dioldicitrat, 2-Ethylhexan-1,3-diolditartrat, 2-Ethylhexan-1,3-dioldiadipat, 2-Ethylhexan-1,4-diolmonolactat, 2-Ethylhexan-1,4-diolmonocitrat, 2-Ethylhexan-1,4-diolmonotartrat, 2-Ethylhexan-1,4-diolmonoadipat, 2-Ethylhexan-1,4-dioldilactat, 2-Ethylhexan-1,4-dioldicitrat, 2-Ethylhexan-1,4-diolditartrat, 2-Ethylhexan-1,4-dioldiadipat, 2-Ethylhexan-1,5-diolmonolactat, 2-Ethylhexan-1,5-diolmonocitrat, 2-Ethylhexan-1,5-diolmonotartrat, 2-Ethylhexan-1,5-diolmonoadipat, 2-Ethylhexan-1,5-dioldilactat, 2-Ethylhexan-1,5-dioldicitrat, 2-Ethylhexan-1,5-diolditartrat, 2-Ethylhexan-1,5-dioldiadipat, 2-Ethylhexan-1,6-diolmonolactat, 2-Ethylhexan-1,6-diolmonocitrat, 2-Ethylhexan-1,6-diolmonotartrat, 2-Ethylhexan-1,6-diolmonoadipat, 2-Ethylhexan-1,6-dioldilactat, 2-Ethylhexan-1,6-dioldicitrat, 2-Ethylhexan-1,6-diolditartrat, 2-Ethylhexan-1,6-dioldiadipat, 1,2-Heptandiolmonoladat, 1,2-Heptandiolmonocitrat, 1,2-Heptandiolmonotartrat, 1,2-Heptandiolmonoadipat, 1,2-Heptandioldiladat, 1,2-Heptandioldicitrat, 1,2-Heptandiolditartrat, 1,2-Heptandioldiadipat, 1,3-Heptandiolmonoladat, 1,3-Heptandiolmonocitrat, 1,3-Heptandiolmonotartrat, 1,3-Heptandiolmonoadipat, 1,3-Heptandioldiladat, 1,3-Heptandioldicitrat, 1,3-Heptandiolditartrat, 1,3-Heptandioldiadipat, 1,4-Heptandiolmonoladat, 1,4-Heptandiolmonocitrat, 1,4-Heptandiolmonotartrat, 1,4-Heptandiolmonoadipat, 1,4-Heptandioldilactat, 1,4-Heptandioldicitrat, 1,4-Heptandiolditartrat, 1,4-Heptandioldiadipat, 1,5-Heptandiolmonolactat, 1,5-Heptandiolmonocitrat, 1,5-Heptandiolmonotartrat, 1,5-Heptandiolmonoadipat, 1,5-Heptandioldilactat, 1,5-Heptandioldicitrat, 1,5-Heptandiolditartrat, 1,5-Heptandioldiadipat, 1,6-Heptandiolmonolactat, 1,6-Heptandiolmonocitrat, 1,6-Heptandiolmonotartrat, 1,6-Heptandiolmonoadipat, 1,6-Heptandioldilactat, 1,6-Heptandioldicitrat, 1,6-Heptandiolditartrat, 1,6-Heptandioldiadipat, 1,7-Heptandiolmonolactat, 1,7-Heptandiolmonocitrat, 1,7-Heptandiolmonotartrat, 1,7-Heptandiolmonoadipat, 1,7-Heptandioldilactat, 1,7-Heptandioldicitrat, 1,7-Heptandiolditartrat, 1,7-Heptandioldiadipat, 1,2-Octandiolmonolactat, 1,2-Octandiolmonocitrat, 1,2-Octandiolmonotartrat, 1,2-Octandiolmonoadipat, 1,2-Octandioldilactat, 1,2-Octandioldicitrat, 1,2-Octandiolditartrat, 1,2-Octandioldiadipat, 1,3-Octandiolmonolactat, 1,3-Octandiolmonocitrat, 1,3-Octandiolmonotartrat, 1,3-Octandiolmonoadipat, 1,3-Octandioldilactat, 1,3-Octandioldicitrat, 1,3-Octandiolditartrat, 1,3-Octandioldiadipat, 1,4-Octandiolmonolactat, 1,4-Octandiolmonocitrat, 1,4-Octandiolmonotartrat, 1,4-Octandiolmonoadipat, 1,4-Octandioldilactat, 1,4-Octandioldicitrat, 1,4-Octandiolditartrat, 1,4-Octandioldiadipat, 1,5-Octandiolmonolactat, 1,5-Octandiolmonocitrat, 1,5-Octandiolmonotartrat, 1,5-Octandiolmonoadipat, 1,5-Octandioldilactat, 1,5-Octandioldicitrat, 1,5-Octandiolditartrat, 1,5-Octandioldiadipat, 1,6-Octandiolmonolactat, 1,6-Octandiolmonocitrat, 1,6-Octandiolmonotartrat, 1,6-Octandiolmonoadipat, 1,6-Octandioldilactat, 1,6-Octandioldicitrat, 1,6-Odandiolditartrat, 1,6-Octandioldiadipat, 1,7-Octandiolmonolactat, 1,7-Octandiolmonocitrat, 1,7-Octandiolmonotartrat, 1,7-Octandiolmonoadipat, 1,7-Octandioldilactat, 1,7-Octandioldicitrat, 1,7-Octandiolditartrat, 1,7-Octandioldiadipat, 1,8-Octandiolmonolactat, 1,8-Octandiolmonocitrat, 1,8-Octandiolmonotartrat, 1,8-Octandiolmonoadipat, 1,8-Octandioldilactat, 1,8-Octandioldicitrat, 1,8-Octandiolditartrat, 1,8-Octandioldiadipat, 2-Methyl-1,3-propandiolmonolactat, 2-Methyl-1,3-propandiolmonocitrat, 2-Methyl-1,3-propandiolmonotartrat, 2-Methyl-1,3-propandiolmonoadipat, 2-Methyl-1,3-propandioldilactat, 2-Methyl-1,3-propandioldicitrat, 2-Methyl-1,3-propandiolditartrat, 2-Methyl-1,3-propandioldiadipat, Dipropylenglycolmonolactat, Dipropylenglycolmonotartrat, Dipropylenglycolmonocitrat, Dipropylenglycolmonoadipat, Dipropylenglycoldilactat, Dipropylenglycolditartrat, Dipropylenglycoldicitrat, Dipropylenglycoldiadipat, Glycerinmonolactat, Glycerinmonotartrat, Glycerinmonocitrat, Glycerinmonoadipat, Glycerindilactat, Glycerinditartrat, Glycerindicitrat, Glycerindiadipat, Glycerintrilactat, Glycerintritartrat, Glycerintricitrat, Glycerintriadipat, Diglycerinmonolactat, Diglycerinmonotartrat, Diglycerinmonocitrat, Diglycerinmonoadipat, Diglycerindilactat, Diglycerinditartrat, Diglycerindicitrat, Diglycerindiadipat, Diglycerintrilactat, Diglycerintritartrat, Diglycerintricitrat, Diglycerintriadipat, Tripropylenglycolmonolactat, Tripropylenglycolmonotartrat, Tripropylenglycolmonocitrat, Tripropylenglycolmonoadipat, Tripropylenglycoldilactat, Tripropylenglycolditartrat, Tripropylenglycoldicitrat, Tripropylenglycoldiadipat, Tripropylenglycoltrilactat, Tripropylenglycoltritartrat, Tripropylenglycoltricitrat, Tripropylenglycoltriadipat, Triglycerinmonolactat, Triglycerinmonotartrat, Triglycerinmonocitrat, Triglycerinmonoadipat, Triglycerindilactat, Triglycerinditartrat, Triglycerindicitrat, Triglycerindiadipat, Triglycerintrilactat, Triglycerintritartrat, Triglycerintricitrat, Triglycerintriadipat, 1,2,6-Hexantriolmonolactat, 1,2,6-Hexantriolmonotartrat, 1,2,6-Hexantriolmonocitrat, 1,2,6-Hexantriolmonoadipat, 1,2,6-Hexantrioldilactat, 1,2,6-Hexantriolditartrat, 1,2,6-Hexantrioldicitrat, 1,2,6-Hexantrioldiadipat, 1,2,6-Hexantrioltrilactat, 1,2,6-Hexantrioltritartrat, 1,2,6-Hexantrioltricitrat, 1,2,6-Hexantrioltriadipat, Trimethylolpropanmonolactat, Trimethylolpropanmonotartrat, Trimethylolpropanmonocitrat, Trimethylolpropanmonoadipat, Trimethylolpropandilactat, Trimethylolpropanditartrat, Trimethylolpropandicitrat, Trimethylolpropandiadipat, Trimethylolpropantrilactat, Trimethylolpropantritartrat, Trimethylolpropantricitrat, Trimethylolpropantriadipat, Trimethylolethanmonolactat, Trimethylolethanmonotartrat, Trimethylolethanmonocitrat, Trimethylolethanmonoadipat, Trimethylolethandilactat, Trimethylolethanditartrat, Trimethylolethandicitrat, Trimethylolethandiadipat, Trimethylolethantrilactat, Trimethylolethantritartrat, Trimethylolethantricitrat und Trimethylolethantriadipat, sowie Mischungen hiervon.

Weitere erfindungsgemäß bevorzugte nichtflüchtige Nichtsiliconöle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltriisostearin und die Handelsprodukte Estol^{®} GTEH 3609 (Uniqema) oder Myritol^{®} GTEH (Cognis) mit verzweigten Fettsäureresten.

Weitere erfindungsgemäß besonders bevorzugte nichtflüchtige Nichtsiliconöle sind ausgewählt aus den symmetrischen, unsymmetrischen oder cyclischen Estern der Kohlensäure mit Fettalkoholen, z. B. Glycerincarbonat, Dicaprylylcarbonat (Cetiol^{®} CC), Di-n-octylcarbonat, Di-n-dodecylcarbonat, Di-(2-ethylhexyl)carbonat oder die Ester gemäß der Lehre der DE 19756454 A. Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂- C₆-Alkanolen.

Es kann erfindungsgemäß außerordentlich bevorzugt sein, Mischungen der vorgenannten Öle einzusetzen, um eine optimale Feinabstimmung der Produkteigenschaften, insbesondere der Stifteigenschaften, wie Stifthärte, Rückstandsverhalten, Abriebeigenschaften oder Wirkstofffreisetzung, zu erzielen.

Weitere erfindungsgemäß bevorzugte Zusammensetzungen sind dadurch gekennzeichnet, dass mindestens ein nichtflüchtiges Nichtsiliconöl in einer Gesamtmenge von 10 - 95 Gew.-%, bevorzugt 15 - 75 Gew.-%, besonders bevorzugt 18 - 50 Gew.-%, außerordentlich bevorzugt 20 - 35 Gew.-%, jeweils bezogen auf die gesamte Zusammensetzung, enthalten ist.

Gemäß einer weiteren, ebenfalls bevorzugten Ausführungsform enthalten die erfindungsgemäßen wasserfreien Antitranspirant-Zusammensetzungen einen geringen Anteil von maximal 2 Gew.-%, bevorzugt maximal 1 Gew.-%, an Cyclomethicone oder sind sogar frei von Cyclomethicone. Als Ersatz für Cyclomethicone sind besonders bevorzugt die C₈-C₁₆-Isoparaffine, insbesondere ausgewählt aus Isononan, Isodecan, Isoundecan, Isododecan, Isotridecan, Isotetradecan, Isopentadecan, und Isohexadecan, sowie Mischungen hiervon. Bevorzugt sind C₁₀-C₁₃-Isoparaffin-Mischungen, insbesondere solche mit einem Dampfdruck bei 20°C von etwa 8 - 400 Pa, bevorzugt 13 - 300 Pa.

Neben dem mindestens einen vorgenannten C₈-C₁₆-Isoparaffin enthalten weitere erfindungsgemäß bevorzugte Antitranspirant-Zusammensetzungen neben 0 bis maximal 2 Gew.-%, bevorzugt maximal 1 Gew.-%, an Cyclomethicone mindestens ein nichtflüchtiges kosmetisches Öl, ausgewählt aus nichtflüchtigen Siliconölen und nichtflüchtigen Nichtsiliconölen. Das mindestens eine nichtflüchtige Öl gleicht den negativen Effekt des flüchtigen Isoparaffins auf das Rückstandsverhalten erfindungsgemäß bevorzugter Antitranspirant-Zusammensetzungen aus. Durch die relativ schnelle Verdunstung der flüchtigen Öle können feste, unlösliche Bestandteile, insbesondere die Antitranspirantwirkstoffe, auf der Haut als unschöner Rückstand sichtbar werden. Diese Rückstände können erfolgreich mit einem nichtflüchtigen Öl maskiert werden. Außerdem können mit einem Gemisch aus nichtflüchtigem und flüchtigem Öl Parameter wie Hautgefühl, Sichtbarkeit des Rückstands und Stabilität der Suspension feinreguliert und besser an die Bedürfnisse der Verbraucher angepasst werden. Hierzu besonders bevorzugte nichtflüchtige Öle sind insbesondere die Esteröle 2-Ethylhexylpalmitat (z. B. Cegesoft^{®} C 24), Hexyldecyllaurat, 2-Ethylhexylstearat, Isopropylmyristat, Isopropylpalmitat und 2-Ethylhexyllaurat, die Benzoesäureester von linearen oder verzweigten C₈₋₂₂-Alkanolen, insbesondere das Handelsprodukt Finsolv^{®} TN (C₁₂-C₁₅-Alkylbenzoat), C₁₂-C₁₅-Alkyllactat und Di-C₁₂-C₁₃-Alkylmalat geeignet. Weiterhin kann es besonders bevorzugt sein, erfindungsgemäße wasserfreie Antitranspirant-Zusammensetzungen ohne Cyclomethicone und ohne flüchtige lineare Siliconöle zu formulieren. Auch hierzu sind besonders bevorzugt die Esteröle 2-Ethylhexylpalmitat (z. B. Cegesoft^{®} C 24), Hexyldecyllaurat, 2-Ethylhexylstearat, Isopropylmyristat, Isopropylpalmitat und 2-Ethylhexyllaurat, die Benzoesäureester von linearen oder verzweigten C₈₋₂₂-Alkanolen, insbesondere das Handelsprodukt Finsolv^{®} TN (C₁₂-C1₅₋Alkylbenzoat), C₁₂-C₁₅-Alkyllactat und Di-C₁₂-C₁₃-Alkylmalat geeignet. Erfindungsgemäß besonders bevorzugte Ölmischungen mit 0 bis maximal 2 Gew.-%, bevorzugt 0 bis maximal 1 Gew.-%, an Cyclomethicone sind 2-Ethylhexylpalmitat/Isodecan/Isoundecan/Isododecan/Isotridecan, Hexyldecyllaurat/Isodecan/Isoundecan/Isododecan/Isotridecan, 2-Ethylhexylstearat/Isodecan/Isoundecan/Isododecan/Isotridecan, Isopropylmyristat/Isodecan/Isoundecan/Isododecan/Isotridecan, Isopropylpalmitat/Isononan/Isodecanlisoundecan/Isododecan/Isotridecan, 2-Ethylhexyllaurat/Isodecan/Isoundecan/Isododecan/Isotridecan, C₁₂-C₁₅-Alkyllactat/Isodecan/Isoundecan/Isododecan/Isotridecan, C₁₂-C₁₅-Alkylbenzoat/Isodecan/Isoundecan/Isododecan/ Isotridecan und Di-C₁₂-C₁₃-Alkylmalat/Isodecan/Isoundecan/ Isododecan/Isotridecan.

In bevorzugten Ölmischungen mit 0 bis maximal 2 Gew.-%, bevorzugt 0 bis maximal 1 Gew.-%, an Cyclomethicone sind die beiden Öltypen (Ester/C₈-C₁₆-Isoparaffin) in etwa gleichen Gewichtsanteilen enthalten, das heißt, in Gewichtsverhältnissen Ester/C₈-C₁₆-Isoparaffin von 0,9 bis 1,2, bevorzugt 1 bis 1,1.

In anderen bevorzugten Ausführungsformen der Erfindung liegt ein Überschuss an C₈-C₁₆-Isoparaffin gegenüber dem/den Ester(n) vor. In diesen Fällen beträgt das Gewichtsverhältnis Ester/ C₈₋₁₆-Isoparaffin bevorzugt 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen Isopropylmyristat/C₈-C₁₆-Isoparaffin in etwa gleichen Gewichtsanteilen enthalten, das heißt, in Gewichtsverhältnissen Ester/C₈-C₁₆-Isoparaffin von 0,9 bis 1,2, bevorzugt 1 bis 1,1. Weitere bevorzugte Gewichtsverhältnisse Isopropylmyristat/C₈₋₁₆-Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen Isopropylpalmitat/C₈-G₁₆-Isoparaffin in etwa gleichen Gewichtsanteilen enthalten, das heißt, in Gewichtsverhältnissen Ester/C₈-C₁₆-Isoparaffin von 0,9 bis 1,2, bevorzugt 1 bis 1,1. Weitere bevorzugte Gewichtsverhältnisse Isopropylpalmitat/C₈₋₁₆-Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen 2-Ethylhexylpalmitat/C₈-C₁₆-Isoparaffin in etwa gleichen Gewichtsanteilen enthalten, das heißt, in Gewichtsverhältnissen Ester/C₈-C₁₆-Isoparaffin von 0,9 bis 1,2, bevorzugt 1 bis 1,1. Weitere bevorzugte Gewichtsverhältnisse 2-Ethylhexylpalmitat/C₈₋₁₆-Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen C₁₂-C₁₅-Alkylbenzoat/C₈-C₁₆-Isoparaffin in etwa gleichen Gewichtsanteilen enthalten, das heißt, in Gewichtsverhältnissen Ester/C₈-C₁₆-Isoparaffin von 0,9 bis 1,2, bevorzugt 1 bis 1,1. Weitere bevorzugte Gewichtsverhältnisse C₁₂-C₁₅-Alkylbenzoat/C₈₋₁₆-Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen Ester/C₁₀-C₁₃-Isoparaffin in etwa gleichen Gewichtsanteilen enthalten, das heißt, in Gewichtsverhältnissen Ester/C₁₀-C₁₃-Isoparaffin von 0,9 bis 1,2, bevorzugt 1 bis 1,1. Weitere bevorzugte Gewichtsverhältnisse Ester/C₁₀₋₁₃-Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen Isopropylmyristat/C₁₀-C₁₃-Isoparaffin in etwa gleichen Gewichtsanteilen enthalten, das heißt, in Gewichtsverhältnissen Ester/C₁₀-C₁₃-Isoparaffin von 0,9 bis 1,2, bevorzugt 1 bis 1,1. Weitere bevorzugte Gewichtsverhältnisse Isopropylmyristat/C₁₀₋₁₃-Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen Isopropylpalmitat/C₁₀-C₁₃-Isoparaffin in etwa gleichen Gewichtsanteilen enthalten, das heißt, in Gewichtsverhältnissen Ester/C₁₀-C₁₃-Isoparaffin von 0,9 bis 1,2, bevorzugt 1 bis 1,1. Weitere bevorzugte Gewichtsverhältnisse Isopropylpalmitat/C₁₀₋₁₃-Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen 2-Ethylhexylpalmitat/C₁₀-C₁₃-Isoparaffin in etwa gleichen Gewichtsanteilen enthalten, das heißt, in Gewichtsverhältnissen Ester/C₁₀-C₁₃-Isoparaffin von 0,9 bis 1,2, bevorzugt 1 bis 1,1. Weitere bevorzugte Gewichtsverhältnisse 2-Ethylhexylpalmitat/C₁₀₋₁₃-Isoparaffin liegen im Bereich von 0,1 bis 0,8, besonders bevorzugt 0,3 bis 0,6, außerordentlich bevorzugt 0,4 bis 0,5.

In weiteren bevorzugten Ölmischungen mit 0 bis maximal 2 Gew.-%, bevorzugt 0 bis maximal 1 Gew.-%, an Cyclomethicone sind die beiden Öltypen C₁₂-C₁₅-Alkylbenzoat/C₁₀-C₁₃-Isoparaffin in etwa gleichen Gewichtsanteilen enthalten, das heißt, in Gewichtsverhältnissen Ester/C₁₀-C₁₃-Isoparaffin von 0,9 bis 1,2, bevorzugt 1 bis 1,1. Weitere bevorzugte Gewichtsverhältnisse C₁₂-C₁₅-Alkylbenzoat/C₁₀₋₁₃-Isoparaffin liegen im Bereich von 0,1 bis 0,8, besonders bevorzugt 0,3 bis 0,6, außerordentlich bevorzugt 0,4 bis 0,5.

Besonders bevorzugte erfindungsgemäße Zusammensetzungen sind dadurch gekennzeichnet, dass das unter Normalbedingungen flüssige Träger-Öl b) eine Mischung b)i) + b)ii) + b)iii) aus b)i) einem Esteröl, ausgewählt aus 2-Ethylhexylpalmitat, Isopropylmyristat und Isopropylpalmitat, und b)ii) mindestens einem Isoparaffinöl, ausgewählt aus Isodecan, Isoundecan, Isododecan, Isotridecan, Isotetradecan, Isopentadecan, Isohexadecan und Isoeicosan, und b)iii) 0 - maximal 1 Gew.-% Cyclomethicone umfasst.

Weitere besonders bevorzugte erfindungsgemäße Zusammensetzungen sind dadurch gekennzeichnet, dass das unter Normalbedingungen flüssige Träger-Öl b) aus einer Mischung b)i) + b)ii) + b)iii) aus b)i) einem Esteröl, ausgewählt aus 2-Ethylhexylpalmitat, Isopropylmyristat und Isopropylpalmitat, und b)ii) mindestens einem Isoparaffinöl, ausgewählt aus Isodecan, Isoundecan, Isododecan, Isotridecan, Isotetradecan, Isopentadecan, Isohexadecan und Isoeicosan, und b)iii) 0 bis maximal 1 Gew.-% Cyclomethicone besteht.

Weitere bevorzugte erfindungsgemäße Zusammensetzungen sind dadurch gekennzeichnet, dass das unter Normalbedingungen flüssige Trägeröl ausgewählt ist aus einer Mischung von Triethylcitrat und mindestens einem weiteren unter Normalbedingungen flüssigen kosmetischen Öl als Träger, wobei der Gewichtsanteil von Triethylcitrat an der Gesamtmenge an Ölen, bezogen auf die gesamte Zusammensetzung, 13 - 50 Gew.-% beträgt und 0 bis weniger als 1 Gew.-% Cyclomethicone, bezogen auf das Gewicht der Zusammensetzung, enthalten ist. Bevorzugt beträgt der Gewichtsanteil von Triethylcitrat am Gesamtölgehalt 12 - 40 Gew.-%, besonders bevorzugt 16-35 Gew.-%, außerordentlich bevorzugt 20 - 30 Gew.-%.

Erfindungsgemäß besonders bevorzugte Ölmischungen sind Triethylcitrat/2-Ethylhexylpalmitat/ Isodecan/Isoundecan/Isododecan/Isotridecan, Triethyleitrat/Hexyldecyllaurat/Isodecan/Isoundecan/Isododecan/Isotridecan, Triethylcitrat/2-Ethylhexylstearat/Isodecan/Isoundecan/Isododecan/ Isotridecan, Triethylcitrat/Isopropylmyristat/Isodecan/Isoundecan/Isododecan/Isotridecan, Triethylcitrat/Isopropylpalmitat/Isononan/Isodecan/Isoundecan/Isododecan/Isotridecan, Triethylcitrat/ 2-Ethylhexyllaurat/Isodecan/Isoundecan/Isododecan/Isotridecan, Triethylcitrat/C₁₂-C₁₅-Alkyllactat/ Isodecan/Isoundecan/Isododecan/Isotridecan, Triethylcitrat/C₁₂-C₁₅-Alkylbenzoat/Isodecan/Isoundecan/Isododecan/Isotridecan und Triethylcitrat/Di-C₁₂-C₁₃-Alkylmalat/Isodecan/Isoundecan/Isododecan/Isotridecan.

In bevorzugten Ölmischungen sind alle drei Öltypen (Triethylcitrat/Ester/C₈-C₁₆-Isoparaffin) in gleichen Gewichtsanteilen enthalten. Weitere bevorzugte Gewichtsverhältnisse Triethylcitrat/ Ester/C₈₋₁₆-Isoparaffin sind (1-1,3) : (0,6-1) : (1-3). Weitere bevorzugte Gewichtsverhältnisse Triethylcitrat/Ester/C₈-C₁₆-Isoparaffin sind (1-1,3) : 1 : (1-1,5). Weitere bevorzugte Gewichtsverhältnisse Triethylcitrat/Ester/C₈-C₁₆-isoparaffin sind (1-1,3) : (0,6 - 0,9) : (2,5 - 3), insbesondere 1 : 0,8 : 3.

In bevorzugten Ölmischungen sind alle drei Öltypen (Triethylcitrat/Isopropylmyristat/C₈-C₁₆-Isoparaffin) in gleichen Gewichtsanteilen enthalten. Weitere bevorzugte Gewichtsverhältnisse Triethylcitrat/Isopropylmyristat/C₈₋₁₆-Isoparaffin sind (1-1,3) : (0,6-1) : (1-3). Weitere bevorzugte Gewichtsverhältnisse Triethylcitrat/Isopropylmyristat/C₈-C₁₆-Isoparaffin sind (1-1,3) : 1 : (1-1,5). Weitere bevorzugte Gewichtsverhältnisse Triethylcitrat/Isopropylmyristat/C₈-C₁₆-Isoparaffin sind (1-1,3) : (0,6 - 0,9) : (2,5 - 3), insbesondere 1 : 0,8 : 3.

In bevorzugten Ölmischungen sind alle drei Öltypen (Triethylcitrat/Isopropylpalmitat/C₈-C₁₆-Isoparaffin) in gleichen Gewichtsanteilen enthalten. Weitere bevorzugte Gewichtsverhältnisse Triethylcitrat/Isopropylpalmitat/C₈₋₁₆-Isoparaffin sind (1-1,3) : (0,6-1) : (1-3). Weitere bevorzugte Gewichtsverhältnisse Triethylcitrat/Isopropylpalmitat/C₈-C₁₆-Isoparaffin sind (1-1,3) : 1 : (1-1,5). Weitere bevorzugte Gewichtsverhältnisse Triethylcitrat/Isopropylpalmitat/C₈-C₁₆-Isoparaffin sind (1-1,3) : (0,6 - 0,9): (2,5 - 3), insbesondere 1 : 0,8 : 3.

In bevorzugten Ölmischungen sind alle drei Öltypen (Triethylcitrat/C₁₂-C₁₅-Alkylbenzoat/C₈-C₁₆-Isoparaffin) in gleichen Gewichtsanteilen enthalten. Weitere bevorzugte Gewichtsverhältnisse Triethylcitrat/C₁₂-C₁₅-Alkylbenzoat/C₈₋₁₆-Isoparaffin sind (1-1,3) : (0,6-1) : (1-3). Weitere bevorzugte Gewichtsverhältnisse Triethylcitrat/C₁₂-C₁₅-Alkylbenzoat/C₈-C₁₆-Isoparaffin sind (1-1,3) : 1 : (1-1,5). Weitere bevorzugte Gewichtsverhältnisse Triethylcitrat/C₁₂-C₁₅-Alkylbenzoat/C₈-C₁₆-Isoparaffin sind (1-1,3): (0,6 - 0,9): (2,5 - 3), insbesondere 1 : 0,8 : 3.

In weiteren bevorzugten Ölmischungen sind alle drei Öltypen (Triethylcitrat/Ester/C₁₀-C₁₃-Isoparaffin) in gleichen Gewichtsanteilen enthalten. Weitere bevorzugte Gewichtsverhältnisse Triethylcitrat/Ester/C₁₀₋₁₃-Isoparaffin sind (1-1,3): (0,6-1) : (1-3). Weitere bevorzugte Gewichtsverhältnisse Triethylcitrat/Ester/C₁₀-C₁₃-Isoparaffin sind (1-1,3) : 1 : (1-1,5). Weitere bevorzugte Gewichtsverhältnisse Triethylcitrat/Ester/ C₁₀-C₁₃-Isoparaffin sind (1-1,3) : (0,6 - 0,9) : (2,5 - 3), insbesondere 1 : 0,8 : 3.

In bevorzugten Ölmischungen sind alle drei Öltypen (Triethylcitrat/Isopropylmyristat/C₁₀-C₁₃-Isoparaffin) in gleichen Gewichtsanteilen enthalten. Weitere bevorzugte Gewichtsverhältnisse Triethylcitrat/Isopropylmyristat/C₁₀₋₁₃-Isoparaffin sind (1-1,3) : (0,6-1) : (1-3). Weitere bevorzugte Gewichtsverhältnisse Triethylcitrat/Isopropylmyristat/C₁₀-C₁₃-Isoparaffin sind (1-1,3) : 1 : (1-1,5). Weitere bevorzugte Gewichtsverhältnisse Triethylcitrat/Isopropylmyristat/C₁₀-C₁₃-Isoparaffin sind (1-1,3) : (0,6 - 0,9): (2,5 - 3), insbesondere 1 : 0,8 : 3.

In bevorzugten Ölmischungen sind alle drei Öltypen (Triethylcitrat/Isopropylpalmitat/C₁₀-C₁₃-Isoparaffin) in gleichen Gewichtsanteilen enthalten. Weitere bevorzugte Gewichtsverhältnisse Triethylcitrat/Isopropylpalmitat/C₁₀₋₁₃-Isoparaffin sind (1-1,3) : (0,6-1) : (1-3). Weitere bevorzugte Gewichtsverhältnisse Triethylcitrat/Isopropylpalmitat/C₁₀-C₁₃-Isoparaffin sind (1-1,3) : 1 : (1-1,5). Weitere bevorzugte Gewichtsverhältnisse Triethylcitrat/Isopropylpalmitat/C₁₀-C₁₃-Isoparaffin sind (1-1,3): (0,6 - 0,9) : (2,5 - 3), insbesondere 1 : 0,8 : 3.

In bevorzugten Ölmischungen sind alle drei Öltypen (Triethylcitrat/C₁₂-C₁₅-Alkylbenzoat/C₁₀-C₁₃-Isoparaffin) in gleichen Gewichtsanteilen enthalten. Weitere bevorzugte Gewichtsverhältnisse Triethylcitrat/C₁₂-C₁₅-Alkylbenzoat/C₁₀₋₁₃-Isoparaffin sind (1-1,3) : (0,6-1) : (1-3). Weitere bevorzugte Gewichtsverhältnisse Triethylcitrat/C₁₂-C₁₅-Alkylbenzoat/C₁₀-C₁₃-Isoparaffin sind (1-1,3): 1 : (1-1,5). Weitere bevorzugte Gewichtsverhältnisse Triethylcitrat/C₁₂-C₁₅-Alkylbenzoat/C₁₀-C₁₃-Isoparaffin sind (1-1,3): (0,6 - 0,9): (2,5 - 3), insbesondere 1 : 0,8 : 3.

Es kann erfindungsgemäß außerordentlich bevorzugt sein, Mischungen der vorgenannten Öle einzusetzen, um eine optimale Feinabstimmung der Produkteigenschaften, insbesondere des Rückstandsverhaltens, des Hautgefühls oder der Wirkstofffreisetzung, zu erzielen.

Weitere erfindungsgemäß bevorzugte Zusammensetzungen sind dadurch gekennzeichnet, dass das mindestens eine unter Normalbedingungen flüssige Träger-Öl b) ausgewählt ist aus flüchtigen cyclischen oder linearen Siliconölen, nichtflüchtigen höhermolekularen linearen Dimethylpolysiloxanen, Estern von linearen oder verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, die hydroxyliert sein können, Benzoesäureestern von linearen oder verzweigten C₈₋₂₂-Alkanolen, den C₈-C₂₂-Fettalkoholestern einwertiger oder mehrwertiger C₂-C₇-Hydroxycarbonsäuren, den Anlagerungsprodukten von Ethylenoxid und/oder Propylenoxid an ein- oder mehrwertige C₃₋₂₀-Alkanole, flüssigen Paraffinölen, Isoparaffinölen, insbesondere Isodecan, Isoundecan, Isododecan, Isotridecan, Isotetradecan, Isopentadecan, Isohexadecan und Isoeicosan, synthetischen Kohlenwasserstoffen, wie Polyisobuten oder Polydecene, und alicyclischen Kohlenwasserstoffen, verzweigten gesättigten oder ungesättigten Fettalkoholen mit 6 - 30 Kohlenstoffatomen, Mischungen aus Guerbetalkoholen und Guerbetalkoholestern, den symmetrischen, unsymmetrischen oder cyclischen Estern der Kohlensäure mit Fettalkoholen, Triglyceriden von linearen oder verzweigten, gesättigten oder ungesättigten, gegebenenfalls hydroxylierten C₈₋₃₀-Fettsäuren, Dicarbonsäureestern von linearen oder verzweigten C₂-C₁₀-Alkanolen, Di-n-alkylethern mit insgesamt 12 bis 36, insbesondere 12 bis 24 C-Atomen, sowie Mischungen der vorgenannten Öle.

Weitere erfindungsgemäß bevorzugte Zusammensetzungen, insbesondere solche in Form von Stiften und Soft Solids, sind dadurch gekennzeichnet, dass sie mindestens eine unter Normalbedingungen feste Fettkomponente mit einem Schmelzpunkt von mehr als 50 -120 °C enthalten. Bevorzugte Fettkomponenten mit einem Schmelzpunkt von > 50 - 120 °C sind ausgewählt aus Wachsen. Generell sind Wachse von fester bis brüchig harter Konsistenz, grob bis feinkristallin, durchscheinend bis opak, jedoch nicht glasartig, und schmelzen oberhalb von 50 °C ohne Zersetzung. Sie sind schon wenig oberhalb des Schmelzpunktes niedrigviskos und zeigen eine stark temperaturabhängige Konsistenz und Löslichkeit.

Erfindungsgemäß bevorzugt sind beispielsweise natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs, Schellackwachs und Walrat. Im Sinne der Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, einsetzbar. Zu den synthetischen Wachsen, die ebenfalls erfindungsgemäß bevorzugt sind, zählen beispielsweise Polyalkylenwachse, insbesondere Polyethylenwachse, und Polyethylenglycolwachse, C₂₀-C₄₀-Dialkylester von Dimersäuren, C₃₀₋₅₀-Alkylbienenwachs sowie Alkyl- und Alkylarylester von Dimerfettsäuren.

Eine besonders bevorzugte Wachskomponente ist ausgewählt aus mindestens einem Ester aus einem gesättigten, einwertigen C₁₆-C₆₀-Alkohol und einer gesättigten C₈-C₃₆-Monocarbonsäure. Erfindungsgemäß zählen hierzu auch Lactide, die cyclischen Doppelester von α-Hydroxycarbonsäuren der entsprechenden Kettenlänge. Ester aus Fettsäuren und langkettigen Alkoholen haben sich für die erfindungsgemäß bevorzugten Zusammensetzungen als besonders vorteilhaft erwiesen, weil sie ausgezeichnete sensorische Eigenschaften und - den Stiften - eine hohe Stabilität verleihen. Die Ester setzen sich aus gesättigten, verzweigten oder unverzweigten Monocarbonsäuren und gesättigten, verzweigten oder unverzweigten einwertigen Alkoholen zusammen. Auch Ester aus aromatischen Carbonsäuren bzw. Hydroxycarbonsäuren (z. B. 12-Hydroxystearinsäure) und gesättigten, verzweigten oder unverzweigten Alkoholen sind erfindungsgemäß einsetzbar, sofern die Wachskomponente einen Schmelzpunkt > 50°C hat. Besonders bevorzugt ist, die Wachskomponenten zu wählen aus der Gruppe der Ester aus gesättigten, verzweigten oder unverzweigten Alkancarbonsäuren einer Kettenlänge von 12 bis 24 C-Atomen und den gesättigten, verzweigten oder unverzweigten Alkoholen einer Kettenlänge von 16 bis 50 C-Atomen, die einen Schmelzpunkt > 50°C haben.

Insbesondere können als Wachskomponente C₁₆₋₃₆-Alkylstearate und C₁₈₋₃₈-Alkylhydroxystearoylstearate, C₂₀₋₄₀-Alkylerucate sowie Cetearylbehenat bevorzugt sein. Das Wachs oder die Wachskomponenten weisen einen Schmelzpunkt > 50°C. bevorzugt > 60°C, auf.

Eine besonders bevorzugte Ausführungsform der Erfindung enthält als Wachskomponente ein C₂₀-C₄₀-Alkylstearat. Dieser Ester ist unter den Namen Kesterwachs^{®} K82H oder Kesterwachs^{®} K80H bekannt und wird von Koster Keunen Inc. vertrieben. Es handelt sich um die synthetische Nachahmung der Monoesterfraktion des Bienenwachses und zeichnet sich durch seine Härte, seine Ölgelierfähigkeit und seine breite Kompatibilität mit Lipidkomponenten aus. Kesterwachs bietet den Vorteil, dass es auch bei geringen Konzentrationen eine exzellente Ölgelierfähigkeit aufweist und so die Stift- oder Soft Solid-Masse nicht zu schwer macht und einen samtigen Abrieb ermöglicht. Eine weitere besonders bevorzugte Ausführungsform der Erfindung enthält als Wachskomponente Cetearylbehenat, d. h. Mischungen aus Cetylbehenat und Stearylbehenat. Dieser Ester ist unter dem Namen Kesterwachs^{®} K62 bekannt und wird von Koster Keunen Inc. vertrieben.

Weitere bevorzugte Wachskomponenten mit einem Schmelzpunkt > 50°C sind die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 50 °C aufweisen, beispielsweise bevorzugt C₁₈ - C₃₆ Acid Triglyceride (Syncrowax^{®} HGL-C). Erfindungsgemäß ist als Wachskomponente hydriertes Rizinusöl, erhältlich z. B. als Handelsprodukt Cutina^{®} HR, besonders bevorzugt.

Weitere bevorzugte Wachskomponenten mit einem Schmelzpunkt > 50°C sind die gesättigten linearen C₁₄ - C₃₆-Carbonsäuren, insbesondere Myristinsäure, Palmitinsäure, Stearinsäure und Behensäure sowie Mischungen dieser Verbindungen, z. B. Syncrowax^{®} AW 1C (C₁₈ - C₃₆-Fettsäuren) oder Cutina^{®} FS 45 (Palmitin- und Stearinsäure).

Bevorzugte erfindungsgemäße Zusammensetzungen sind dadurch gekennzeichnet, dass die Wachskomponente als Bestandteil des erfindungsgemäßen Trägers ausgewählt ist aus Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat, Glycerintriestern von gesättigten linearen C₁₂ - C₃₀-Carbonsäuren, die hydroxyliert sein können, Candelillawachs, Carnaubawachs, Bienenwachs, gesättigten linearen C₁₄ - C₃₆-Carbonsäuren sowie Mischungen der vorgenannten Substanzen. Besonders bevorzugte Wachskomponenten-Mischungen sind ausgewählt aus Mischungen von Cetylbehenat, Stearylbehenat, gehärtetem Rizinusöl, Palmitinsäure und Stearinsäure. Weitere besonders bevorzugte Wachskomponenten-Mischungen sind ausgewählt aus Mischungen von C₂₀-C₄₀-Alkylstearat, gehärtetem Rizinusöl, Palmitinsäure und Stearinsäure.

Besonders bevorzugte erfindungsgemäße Zusammensetzungen sind dadurch gekennzeichnet, dass die Wachskomponente als Bestandteil des erfindungsgemäßen Trägers ausgewählt ist aus Mischungen von Estern aus einem gesättigten, einwertigen C₁₆-C₆₀-Alkanol und einer gesättigten C₈-C₃₈-Monocarbonsäure, insbesondere C₂₀-C₄₀-Alkylstearat, Glycerintriestern von gesättigten linearen C₁₂ - C₃₀-Carbonsäuren, die hydroxyliert sein können, insbesondere hydriertem Rizinusöl, und gesättigten linearen C₁₄ - C₃₆-Carbonsäuren, insbesondere Palmitinsäure und Stearinsäure.

Weitere bevorzugte erfindungsgemäße Zusammensetzungen sind dadurch gekennzeichnet, dass die Wachskomponente/n als Bestandteil des erfindungsgemäßen Trägers in einer Gesamtmenge von 1 - 10 Gew.-%, bevorzugt 1,5 - 8 Gew.-%, besonders bevorzugt 2 - 6 Gew.-%, außerordentlich bevorzugt 3 - 5 Gew.-%, bezogen auf die Gesamtzusammensetzung, enthalten ist.

Weitere bevorzugte erfindungsgemäße Zusammensetzungen sind dadurch gekennzeichnet, dass mindestens eine Wachskomponente mit einem Schmelzpunkt im Bereich von 25 - 50°C, ausgewählt aus Kokosfettsäureglycerinmono-, -di- und -triestem, Butyrospermum Parkii (Shea Butter) und Estern von gesättigten, einwertigen C₈-C₁₈-Alkoholen mit gesättigten C₁₂-C₁₈-Monocarbonsäuren sowie Mischungen dieser Substanzen, enthalten ist. Diese niedriger schmelzenden Wachskomponenten ermöglichen eine Konsistenzoptimierung des Produktes und eine Minimierung der sichtbaren Rückstände auf der Haut. Besonders bevorzugt sind Handelsprodukte mit der INCI-Bezeichnung Cocoglycerides, besonders bevorzugt ein Gemisch aus C₁₂-C₁₈-Mono-, Di- und Triglyceriden, das im Bereich von 30 - 32°C schmilzt, wie es beispielsweise unter dem Handelsnamen Novata^{®} AB von Cognis erhältlich ist, sowie die Produkte der Softisan-Reihe (Sasol Germany GmbH) mit der INCI-Bezeichnung Hydrogenated Cocoglycerides, insbesondere Softisan 100, 133, 134, 138, 142. Weitere bevorzugte Ester von gesättigten, einwertigen C₁₂-C₁₈-Alkoholen mit gesättigten C₁₂-C₁₈-Monocarbonsäuren sind Stearyllaurat, Cetearytstearat (z. B. Crodamol^{®} CSS), Stearylstearat (z. B Estol 3706), Cetylpalmitat (z. B. Cutina^{®} CP, Schmelzpunkt: 46 - 50 °C) und Myristylmyristat (z. B. Cetiol^{®} MM, Schmelzpunkt: 38 - 42 °C).

Weitere bevorzugte erfindungsgemäße Zusammensetzungen sind dadurch gekennzeichnet, dass mindestens eine Wachskomponente mit einem Schmelzpunkt im Bereich von 25 - 50°C in einer Gesamtmenge von 0,01 bis 10 Gew.-%, bevorzugt 0,5 - 8 Gew.-%, besonders bevorzugt 1 - 7,5 Gew.-% und außerordentlich bevorzugt 1,8 - 7 Gew.-%, weiterhin bevorzugt 1,9, 2,0, 2,1, 2,2, 2,3, 2,4, 2,5, 3, 3,5, 4, 4,5, 5, 5,5, 6 und 6,5 Gew.-%, bezogen auf die Gesamtzusammensetzung, enthalten ist.

Weitere bevorzugte erfindungsgemäße Zusammensetzungen sind dadurch gekennzeichnet, dass sie, beispielsweise zur Verbesserung der Konsistenz und der sensorischen Eigenschaften, mindestens einen festen, wasserunlöslichen teilchenförmigen Füllstoff enthalten. In einer außerordentlich bevorzugten Ausführungsform ist dieser Füllstoff ausgewählt aus gegebenenfalls modifizierten Stärken (z. B. aus Mais, Reis, Kartoffeln) und Stärkederivaten, die gewünschtenfalls vorverkleistert sind, Siliciumdioxid, Kieselsäuren, z. B. Aerosil^{®}-Typen, sphärischen Polyalkylsesquisiloxan-Partikeln (insbesondere Aerosil^{®} R972 und Aerosil^{®} 200V von Degussa), Kieselgelen, Talkum, Kaolin, Magnesiumaluminiumsilikaten, Bornitrid, Lactoglobulinderivaten, z. B. Natrium-C₈₋₁₆-Isoalkylsuccinyllactoglobulinsulfonat, von Brooks Industries erhältlich als Handelsprodukt Biopol^{®} OE, Glaspulvern, Polymerpulvern, insbesondere aus Polyolefinen, Polycarbonaten, Polyurethanen, Polyamiden, z. B. Nylon, Polyestern, Polystyrolen, Polyacrylaten, (Meth)acrylat- oder (Meth)acrylat-Vinyliden-Copolymeren, die vernetzt sein können, oder Siliconen, sowie Mischungen dieser Substanzen.

Polymerpulver auf Basis eines Polymethacrylat-Copolymers sind z. B. als Handelsprodukt Polytrap^{®} 6603 (Dow Corning) erhältlich. Andere Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere Polymerpulver, die sich als erfindungsgemäß bevorzugte Füllstoffe eignen, sind z. B. Polymethacrylate (Micropearl^{®} M von SEPPIC oder Plastic Powder A von NIKKOL), Styrol-Divinylbenzol-Copolymeren (Plastic Powder FP von NIKKOL), Polyethylen- und Polypropylen-Pulver (ACCUREL^{®} EP 400 von AKZO) oder auch Siliconpolymere (Silicone Powder X2-1605 von Dow Corning).

Bevorzugte erfindungsgemäße Zusammensetzungen sind dadurch gekennzeichnet, dass sie mindestens einen festen, wasserunlöslichen teilchenförmigen Füllstoff in einer Gesamtmenge von 1 bis 99 Gew.-%, bevorzugt 2 - 90 Gew.-%, besonders bevorzugt 3 -15 Gew.-%, außerordentlich bevorzugt 4, 5, 6, 7, 8, 9, 10, 11, 12, 13 und 14 Gew.-%, jeweils bezogen auf die Gesamtzusammensetzung, enthalten.

Weitere erfindungsgemäß bevorzugte Zusammensetzungen mindestens einen Öl-in-Wasser-Emulgator enthalten. Bevorzugte Öl-Wasser-Emulgatoren weisen einen HLB-Wert von mehr als 7 auf. Weitere bevorzugte Öl-in-Wasser-Emulgatoren sind nichtionisch. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seiten 913 - 916, aufgelistet sind. Für ethoxylierte Produkte wird der HLB-Wert nach der Formel HLB = (100 - L) : 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist.

Bei der Auswahl erfindungsgemäß geeigneter nichtionischer Öl-in-Wasser-Emulgatoren ist es besonders bevorzugt, ein Gemisch von nichtionischen Öl-in-Wasser-Emulgatoren einzusetzen, um die Eigenschaften der erfindungsgemäßen Zusammensetzungen, wie Wirkstofffreisetzung oder Abwaschbarkeit, optimal einstellen zu können. Die einzelnen Emulgatorkomponenten liefern dabei einen Anteil zum Gesamt-HLB-Wert oder mittleren HLB-Wert des Öl-in-Wasser-Emulgatorgemisches gemäß ihrem Mengenanteil an der Gesamtmenge der Öl-in-Wasser-Emulgatoren. Die erfindungsgemäßen Zusammensetzungen, insbesondere die Deodorant- oder Antitranspirant-Stifte, können aber in einer anderen bevorzugten Ausführungsform auch nur einen einzigen Öl-in-Wasser-Emulgator, bevorzugt mit einem HLB-Wert im Bereich von 11 - 17, besonders bevorzugt 12 -15 und außerordentlich bevorzugt 13 - 14, enthalten.

Bevorzugte erfindungsgemäße kosmetische Zusammensetzungen sind dadurch gekennzeichnet, dass die nichtionischen Öl-in-Wasser-Emulgatoren ausgewählt sind aus ethoxylierten C₈-C₂₄-Alkanolen mit durchschnittlich 10 - 100 Mol Ethylenoxid pro Mol, ethoxylierten C₈-C₂₄-Carbonsäuren mit durchschnittlich 10 - 100 Mol Ethylenoxid pro Mol, mit durchschnittlich 20 - 100 Mol Ethylenoxid pro Mol ethoxylierten Sorbitanmonoestern von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, Silicon-Copolyolen mit Ethylenoxid-Einheiten oder mit Ethylenoxid- und Propylenoxid-Einheiten, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit n = 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, sofern sie einen HLB-Wert von mehr als 7 aufweisen, sowie Mischungen der vorgenannten Substanzen.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet.

Die ethoxylierten C₈-C₂₄-Carbonsäuren haben die Formel R¹O(CH₂CH₂O)ₙH, wobei R¹O steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 10 -100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-40-monostearat, PEG-50-monostearat, PEG-100-monostearat, PEG-50-monooleat, PEG-100-monooleat, PEG-50-monolaurat und PEG-100-monolaurat.

Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen, insbesondere Ceteth-10, Ceteth-12, Ceteth-20, Ceteth-30, Steareth-10, Steareth-12, Steareth-20, Steareth-30, Ceteareth-10, Ceteareth-12, Ceteareth-20, Ceteareth-30, Laureth-12 und Beheneth-20. Bevorzugte mit durchschnittlich 20 - 100 Mol Ethylenoxid pro Mol ethoxylierte Sorbitanmonoester von linearen gesättigten und ungesättigten C₁₂ - C₃₀-Carbonsäuren, die hydroxyliert sein können, sind ausgewählt aus Polysorbate-20, Polysorbate-40, Polysorbate-60 und Polysorbate-80. Weiterhin werden vorzugsweise C₈ - C₂₂-Alkylmono- und -oligoglycoside eingesetzt. C₈-C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} erhältlich sind, enthalten eine glucosidisch gebundene C₈-C₁₆-Alkylgruppe an einem Oligoglucosidrest, dessen mittlerer Oligomerisationsgrad bei 1 - 2, insbesondere 1,2 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet. Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von mehr als 7 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.

Weitere erfindungsgemäß bevorzugte Öl-in-Wasser-Emulgatoren sind ausgewählt aus Organosiloxan-Oxyalkylen-Copolymeren. Bevorzugte Organosiloxan-Oxyalkylen-Copolymer-O/W-Emulgatoren sind ausgewählt aus Verbindungen der allgemeinen Strukturformeln (I), (11), (III), (IV) und (V), 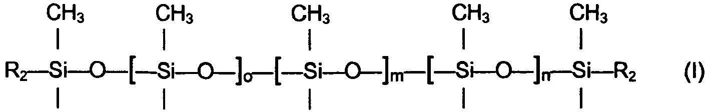 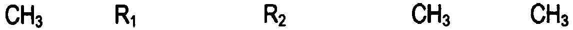 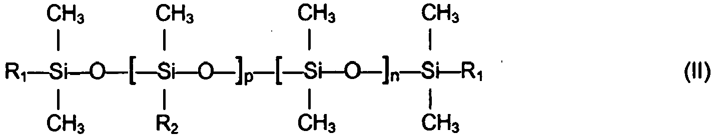 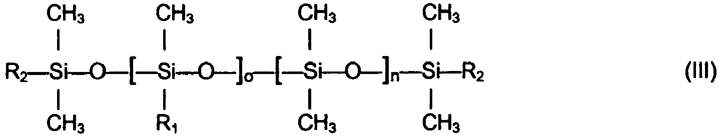 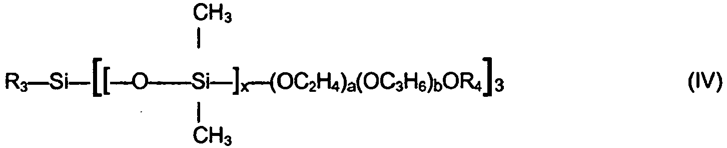 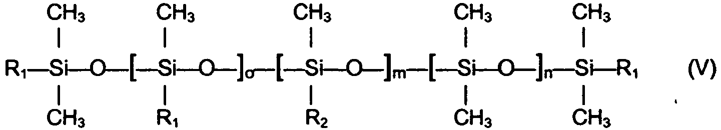 wobei
die Reste R¹ unabhängig voneinander eine lineare oder verzweigte C₁-C₃₀-Alkylgruppe oder eine ggf. substituierte Phenylgruppe darstellen,
die Reste R² unabhängig voneinander die Gruppen -C_{c}H_{2c}-O-(C₂H₄O-)ₐ(C₃H₆O-)_{b}R⁵ oder -C_{c}H_{2c}-O-(C₂H₄O-)ₐR⁵ darstellen,
die Reste R³ und R⁴ unabhängig voneinander eine lineare oder verzweigte C₁-C₁₆-Alkylgruppe und bevorzugt Methylgruppen darstellen,
die Reste R⁵ unabhängig voneinander ein Wasserstoffatom oder eine lineare oder verzweigte C₁-C₁₆-Alkylgruppe und bevorzugt ein Wasserstoffatom oder eine Methylgruppe darstellen,
m eine Zahl von 0 - 20 darstellt,
n eine Zahl von 0 - 500 darstellt,
o eine Zahl von 0 - 20 darstellt,
p eine Zahl von 1 - 50 darstellt,
a eine Zahl von 0 - 50 darstellt,
b eine Zahl von 0 - 50 darstellt,
a + b mindestens 1 sind,
c eine Zahl von 1 - 4, besonders bevorzugt 3, darstellt,
x eine Zahl von 1 -100 darstellt.

Erfindungsgemäß bevorzugte Zusammensetzungen sind dadurch gekennzeichnet, dass mindestens ein Organosiloxan-Oxyalkylen-Copolymer der allgemeinen Strukturformel (II) enthalten ist mit R¹ = Methyl,
R² = -C_{c}H_{2c}-O-(C₂H₄O-)ₐ(C₃H₆O-)_{b}R⁵
mit
a = 18
b= 18
c=3
R⁵ = Methyl,
n = 10 - 500,
p= 10-50.

Ein derartiges Organosiloxan-Oxyalkylen-Copolymer ist beispielsweise unter der Handelsbezeichnung Dow Corning 190 (INCI: PEG/PPG-18/18 Dimethicone) erhältlich.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit
R¹ = Methyl,

R² = -C_{c}H_{2c}-O-(C₂H₄O-)ₐ₍C₃H₆O-)_{b}R⁵
mit
a=12
b=0
c=3
R⁵ = Methyl,
n = 10 - 500,
p = 10 - 50.

Ein derartiges Organosiloxan-Oxyalkylen-Copolymer ist beispielsweise unter der Handelsbezeichnung Dow Corning 193 (INCI: PEG-12 Dimethicone) erhältlich.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit
R¹ = Methyl,
R² = -C_{c}H_{2c}-O-(C₂H₄O-)a(C₃H₆O-)_{b}R⁵
mit
a=7
b=0
c=2
R⁵ = Methyl,
n=0
p=1.

Ein derartiges Organosiloxan-Oxyalkylen-Copolymer ist beispielsweise unter der Handelsbezeichnung Silwet L-77 erhältlich.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit
R¹ = Methyl,
R² = -C_{c}H_{2c}-O-(C₂H₄O-)ₐ(C₃H₆O-)_{b}R⁵
mit
a = 22
b = 24
c = 3
R⁵ = Methyl,
n = 10 - 500,
p= 10-50.

Ein derartiges Organosiloxan-Oxyalkylen-Copolymer ist beispielsweise in einer Mischung mit Cyclomethicone unter der Handelsbezeichnung Mirasil DMCO (INCI: Cyclomethicone, PEG/PPG-22/24 Dimethicone) erhältlich.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit
R¹ = Methyl,
R² = -C_{c}H_{2c}-O-(C₂H₄O-)ₐ(C₃H₆O-)_{b}R⁵
mit
a= 17
b= 18
c=3
R⁵ = Methyl,
n= 10-500,
p= 10-50.

Ein derartiges Organosiloxan-Oxyalkylen-Copolymer ist beispielsweise unter der Handelsbezeichnung Dow Corning Q2-5220 (INCI: PEG/PPG-17/18 Dimethicone) erhältlich.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit
R¹ = Methyl,
R² = -C_{c}H_{2c}-O-(C₂H₄O-)ₐ(C₃H₆O-)_{b}R⁵
mit
a = 20
b = 6
c = 3
R⁵ = Methyl,
n = 10 - 500,
p = 5 - 50.

Ein derartiges Organosiloxan-Oxyalkylen-Copolymer ist beispielsweise unter der Handelsbezeichnung Abil B 88184 (INCI: PEG/PPG-20/6 Dimethicone) erhältlich.

Weitere bevorzugte erfindungsgemäße Zusammensetzungen sind dadurch gekennzeichnet, dass mindestens ein Organosiloxan-Oxyalkylen-Copolymer der allgemeinen Strukturformel (II) enthalten ist mit
R¹ = Methyl,
R² = -C_{c}H_{2c}-O-(C₂H₄O-)ₐ(C₃H₆O-)_{b}R⁵
mit
a = 14
b = 4
c = 3
R⁵ = Methyl,
n = 10 - 500,
p = 5 - 50.

Ein derartiges Organosiloxan-Oxyalkylen-Copolymer ist beispielsweise unter der Handelsbezeichnung Abil B 8851 (INCI: PEG/PPG-14/4 Dimethicone) erhältlich.

Besonders bevorzugte erfindungsgemäße Zusammensetzungen, die als wasserfreier Wachsstift, wasserfreier Soft Solid bzw. wasserfreie Creme konfektioniert sind, sind dadurch gekennzeichnet, dass mindestens ein Öl-in-Wasser-Emulgator, bevorzugt mindestens ein nichtionischer Öl-in-Wasser-Emulgator, in einer Gesamtmenge von 0,5 - 10 Gew.-%, bevorzugt 1-9 Gew.-%, besonders bevorzugt 1,5 - 8,5 Gew.-%, außerordentlich bevorzugt 2 - 8 Gew.-%, weiterhin auch 2,5, 3, 3,5, 4" 4,5, 5, 5,5, 6, 6,5, 7, 7,5, und 8 Gew.-%, bezogen auf die Gesamtzusammensetzung, enthalten ist.

Weitere bevorzugte erfindungsgemäße Zusammensetzungen sind dadurch gekennzeichnet, dass sie als Dibenzylidenalditol-basiertes Gel, insbesondere als Dibenzylidensorbitol-basiertes Gel, formuliert sind. Entsprechende Gele, die normalerweise so fest sind, dass sie als Stift vertrieben werden, sind beispielsweise offenbart in US 5705171, US 5939055, US 5725846, EP 812183 und EP 1269977. Als Träger dient ein wasserfreier mehrwertiger Alkohol mit 3 bis 9 Kohlenstoffatomen, der unter Normalbedingungen flüssig ist. Dazu zählen 1,2-Propylenglycol, 2-Methyl-1,3-propandiol, Glycerin, 1,2-Butylenglycol, 1,3-Butylenglycol und 1,4-Butylenglycol, Pentylenglycole wie 1,2-Pentandiol und 1,5-Pentandiol, Hexandiole wie 1,2-Hexandiol und 1,6-Hexandiol, Hexantriole wie 1,2,6-Hexantriol, 2-Ethyl-2-hydroxymethyl-1,3-propandiol, Diethylenglycol, Triethylenglycol, Dipropylenglycol, Tripropylenglycol, Diglycerin und Triglycerin. Besonders bevorzugt ist 1,2-Propylenglycol. Außerordentlich bevorzugt ist 1,2-Propylenglycol, das 70 - 95 Gew.-%, bevorzugt 75 - 92 Gew.-% des gesamten Stiftgewichts ausmacht. Dieses Lösemittel wird mit einem Dibenzylidenalditol, insbesondere mit Dibenzylidensorbitol (DBS) geliert. Als ähnlich strukturierte Geliermittel ebenfalls geeignet sind Dibenzylidenxylitol und Dibenzylidenribitol, DBS ist dabei bevorzugt. Bevorzugt wird dieses Geliermittel in einer Gesamtmenge von 0,3 - 2 Gew.-%, besonders bevorzugt 0,5 - 1,8 Gew.-%, außerordentlich bevorzugt 0,8 - 1,5 Gew.-%, bezogen auf das Gesamtgewicht der Zusammensetzung, zugesetzt.

Besonders bevorzugt sind die Dibenzylidenalditol-basierten Gele transparent. In einer weiteren bevorzugten Ausführungsform sind weitere Zusatzstoffe, wie insbesondere Antitranspirant- oder Deodorant-Wirkstoffe, Öle, Emulgatoren und natürlich die erfindungsgemäßen Wirkstoffkapseln, so ausgewählt dass sie die Transparenz des Endproduktes nicht beeinträchtigen.

Weiterhin enthalten erfindungsgemäße Dibenzylidenalditol-basierte Gele bevorzugt mindestens einen Antitranspirant-Wirkstoff, besonders bevorzugt in einer Gesamtmenge von 3 - 27 Gew.-%, bevorzugt 5 - 22 Gew.-% und besonders bevorzugt 10 - 20 Gew.-%, jeweils bezogen auf das Gesamtgewicht der kristallwasserfreien Aktivsubstanz (USP) in der Gesamtzusammensetzung. Der mindestens eine Antitranspirant-Wirkstoff liegt besonders bevorzugt gelöst oder solubilisiert in dem Propylenglycol-Träger des Dibenzylidenalditol-basierten Gels vor.

Weiterhin enthalten Dibenzylidenalditol-basierte Gele bevorzugt mindestens ein kosmetisches Öl. Prinzipiell sind alle vorstehend genannten Öle geeignet. Im Hinblick auf das Hautgefühl und die Transparenz sind bevorzugte Öle ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether sowie den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether, PPG-9-Butylether, PPG-10-Butandiol, PPG-15-Stearylether und Glycereth-7-diisononanoat.

Weitere bevorzugte Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)-adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Bevorzugt wird das mindestens eine Öl in einer Gesamtmenge von 0,1 - 5 Gew.-%, besonders bevorzugt 0,5 - 3 Gew.-%, außerordentlich bevorzugt 0,8 - 1,5 Gew.-%, bezogen auf das Gesamtgewicht der Dibenzylidenalditol-gelierten Zusammensetzung, zugesetzt.

Als zusätzlichen Gelbildner enthalten bevorzugte erfindungsgemäße Dibenzylidenalditol-basierte Gele mindestens eine Substanz, ausgewählt aus der Gruppe, umfassend Celluloseether, vor allem Hydroxyalkylcellulosen, insbesondere Hydroxypropylcellulose, Hydroxypropylmethylcellulose, Hydroxyethylcellulose, Carboxymethylcellulose, Cetylhydroxyethylcellulose, Hydroxybutylmethylcellulose, Methylhydroxyethylcellulose, weiterhin Xanthan-Gum, Sclerotium Gum, Succinoglucane, Polygalactomannane, insbesondere Guar-Gums und Johannisbrotkernmehl (Locust Bean Gum), insbesondere Guar-Gum und Locust Bean Gum selbst und die nichtionischen Hydroxyalkylguarderivate und Johannisbrotkernmehl-Derivate, wie Hydroxypropylguar, Carboxymethylhydroxypropylguar, Hydroxypropylmethylguar, Hydroxyethylguar und Carboxymethylguar, weiterhin Pectine, Agar, Carragheen (Carrageenan), Traganth, Gummi arabicum, Karayagummi, Taragummi, Gellan, Gelatine, Casein, Propylenglycolalginat, Alginsäuren und deren Salze, insbesondere Natriumalginat, Kaliumalginat und Calciumalginat. Erfindungsgemäß besonders bevorzugt ist Hydroxypropylcellulose. Dieser zusätzliche Gelbildner ist bevorzugt in einer Gesamtmenge von 0,08 - 1 Gew.-%, bevorzugt 0,1 - 0,5 Gew.-%, besonders bevorzugt 0,2 - 0,4 Gew.-%, außerordentlich bevorzugt 0,3 Gew.-%, jeweils bezogen auf das Gesamtgewicht der Zusammensetzung, enthalten.

In einer weiteren bevorzugten Ausführungsform enthalten die erfindungsgemäßen Dibenzylidenalditol-basierten Gele mindestens einen Komplexbildner. Als Komplexbildner besonders bevorzugt sind wasserfreie Ethylendiamintetraessigsäure (EDTA) und ihre Natriumsalze, insbesondere das Tetranatriumsatz und das Trinatriumsalz, wasserfreie 1,3-Propylendiamintetraessigsäure (PDTA) und ihre Natriumsalze, insbesondere das Tetranatriumsalz und das Trinatriumsalz, wasserfreie Hydroxyethylethylendiamintetraessigsäure (HEDTA) und ihre Natriumsalze, insbesondere das Tetranatriumsalz und das Trinatriumsalz, wasserfreie Diethylentriaminpentaessigsäure (DTPA) und ihre Natriumsalze, insbesondere das Pentanatriumsalz, das Tetranatriumsalz und das Trinatriumsalz, Nitrilotriessigsäure (NTA) und ihre Natriumsalze, β-Alanindiessigsäure und ihre Salze, das Ethanoldiglycindinatriumsalz, das Diethanolglycinnatriumsalz und Phosphonsäuren und deren Salze. Die mindestens eine komplexbildende Substanz ist bevorzugt in einer Gesamtmenge von 0,05 - 2 Gew.-%, besonders bevorzugt 0,1 - 1, außerordentlich bevorzugt 0,2 - 0,5 Gew.-%, bezogen auf das Gesamtgewicht der erfindungsgemäßen Zusammensetzung, enthalten.

In einer weiteren bevorzugten Ausführungsform enthalten die erfindungsgemäßen Dibenzylidenalditol-basierten Gele mindestens einen Öl-in-Wasser-Emulgator. Bevorzugte Öl-in-Wasser-Emulgatoren, insbesondere bevorzugte nichtionische Öl-in-Wasser-Emulgatoren, sind vorstehend bereits beschrieben.

Besonders bevorzugte erfindungsgemäße Dibenzylidenalditol-basierte Gel-Zusammensetzungen sind dadurch gekennzeichnet, dass mindestens ein Öl-in-Wasser-Emulgator, bevorzugt mindestens ein nichtionischer Öl-in-Wasser-Emulgator, in einer Gesamtmenge von 0,1 - 5 Gew.-%, bevorzugt 0,2 - 3 Gew.-%, besonders bevorzugt 0,3 - 2 Gew.-%, außerordentlich bevorzugt 0,4-1 Gew.-%, weiterhin bevorzugt auch 0,5, 0,6, 0,7, 0,8 und 0,9 Gew.-%, bezogen auf die Gesamtzusammensetzung, enthalten ist.

Die Konfektionierung der erfindungsgemäßen Zusammensetzungen richtet sich vorzugsweise nach den Anforderungen der gewünschten Applikation.

Die erfindungsgemäßen Zusammensetzungen liegen bevorzugt als Suspension vor, das heißt, der schweißhemmende Wirkstoff und gegebenenfalls weitere unlösliche Bestandteile sind in einem flüssigen Träger suspendiert.

In einer erfindungsgemäß bevorzugten Ausführungsform sind die erfindungsgemäßen Zusammensetzungen als nicht versprühbare Suspension konfektioniert. Ein solches disperses System sollte vor der Anwendung geschüttelt werden.

Die vorstehend beschriebene Suspension ist in einer anderen bevorzugten Ausführungsform verdickt oder zu einem Stift verfestigt.

Bevorzugte erfindungsgemäße Zusammensetzungen sind z. B. in Rollspendern oder Stiftspendem abgepackt.

Die Konfektionierung der erfindungsgemäßen Zusammensetzungen, die als Nonaerosol appliziert werden, richtet sich vorzugsweise nach den Anforderungen der Applikationsart.

Bevorzugte erfindungsgemäße Zusammensetzungen liegen in fester, halbfester, flüssiger, disperser, wasserfrei emulgierter, suspendierter oder gelförmiger Form vor.

In einer besonders bevorzugten Ausführungsform liegen die erfindungsgemäßen Zusammensetzungen in flüssiger Form vor. Unter den Begriff der Flüssigkeit fallen im Sinne der Erfindung auch jegliche Festkörperdispersionen in Flüssigkeiten. Erfindungsgemäße Zusammensetzungen können auch als Pasten, Salben, Lotionen oder Cremes vorliegen. Feste Zusammensetzungen können beispielsweise als loser Puder, gepresster Puder oder als Stift vorliegen.

Die Applikation kann z.B. im Falle flüssiger Mittel vorzugsweise auch mit einem Roller-Applikator erfolgen, wie er z.B. aus dem Bereich der Deo-Roller bekannt ist. Solche Roller weisen eine in einem Kugelbett gelagerte Kugel auf, die durch Bewegung über eine Oberfläche bewegt werden kann. Dabei nimmt die Kugel etwas von dem zu verteilenden Mittel auf und befördert dieses an die zu behandelnde Oberfläche.

Die erfindungsgemäßen Mittel, vorzugsweise flüssigen Mittel, können auch mehrphasig sein, die Phasen können z.B. horizontal, also übereinander, oder vertikal, also nebeneinander, angeordnet sein. Es kann sich auch um ein disperses System handeln, bei dem z.B. die festen Bestandteile inhomogen in der flüssigen Matrix verteilt sind, so dass ein solches disperses System vor der Anwendung geschüttelt werden sollte.

Antitranspirant-Stifte auf wasserfreier Basis können in gelierter Form vorliegen, wobei die Öl-phase mindestens eine Siliconkomponente enthalten oder aus mindestens einer Siliconkomponente bestehen kann. Weiterhin können die erfindungsgemäßen Zusammensetzungen, die als Antitranspirant-Stifte formuliert sind, auf wasserfreier Fettbasis, auf Basis einer Polyol-in-Öl-Emulsion, auf Basis einer Öl-in-Polyol-Emulsion, auf Basis einer Polyol-Öl-Mehrfach-Emulsion, auf Basis einer Nanoemulsion und auf Basis einer Mikroemulsion vorliegen, wobei die Polyolphase wasserfrei ist. Gelstifte können auf der Basis von Alditolen, insbesondere Dibenzylidensorbitol, N-Acylaminosäureamiden, 12-Hydroxystearinsäure, Polyamiden und Polyamidderivaten, formuliert werden.

Wasserfreie Antitranspirant-Wachsstifte enthalten etwa 30 - 70 Gew.-% an mindestens einem unter Normalbedingungen flüssigen kosmetischen Öl, etwa 15 - 25 Gew.-% einer unter Normalbedingungen festen Fettkomponente, wovon üblicherweise der größte Anteil einen Schmelzpunkt von ca. 50 °C aufweist bzw. üblicherweise aus Fettalkoholen, insbesondere aus Stearylalkohol, aber auch Cetylalkohol und ggf. noch Arachidylalkohol und/oder Behenylalkohol besteht, während ein geringerer Anteil - etwa 0,5 - 5 Gew.-% - aus mindestens einer Fettkomponente mit einem Schmelzpunkt von ca. 55 - 120 °C besteht. Darüber hinaus können 0,5 - 8 Gew.-% mindestens einer Fettkomponente mit einem Schmelzpunkt von ca. 25 - 35 °C enthalten sein. Weiterhin können 0,5 - 30 Gew.-% mindestens eines Füllstoffes enthalten sein, der typischerweise ausgewählt ist aus Talkum, Cellulosepulvern, Stärken und Stärkederivaten. Weiterhin können 0,1 - 10 Gew.-%, bevorzugt 1 - 5 Gew.-%, besonders bevorzugt 2 - 4 Gew.-% mindestens eines Öl-in-Wasser-Emulgators enthalten sein.

Eine erfindungsgemäß bevorzugte Ausführungsform als wasserfreier Antitranspirant-Wachsstift ist durch folgende Inhaltsstoffe gekennzeichnet:
5 - 40 Gew.-%, bevorzugt 10 - 35 Gew.-%, besonders bevorzugt 11 - 28 Gew.-% und außerordentlich bevorzugt 12 - 20 Gew.-% (USP) mindestens eines Antitranspirant-Wirkstoffs,
30 - 70 Gew.-% an mindestens einem unter Normalbedingungen flüssigen kosmetischen Öl, 15 - 32 Gew.-% einer unter Normalbedingungen festen Fettkomponente, wovon mehr als 65 Gew.-%, bevorzugt mehr als 70 Gew.-%, besonders bevorzugt mehr als 80 Gew.-%, jeweils bezogen auf den Gesamtgehalt an unter Normalbedingungen festen Fettkomponenten, aus C₁₆-C₃₀-Fettalkoholen, bevorzugt ausgewählt aus der Gruppe, bestehend aus Stearylalkohol, Cetylalkohol und Mischungen hiervon, besteht, wobei besonders bevorzugt zusätzlich zu Stearylalkohol und/oder Cetylalkohol weiterhin 0,1 - 3 Gew.-%, bezogen auf den gesamten Stift, Arachidylalkohol und/oder Behenylalkohol und/oder mindestens ein C₂₄-C₃₀-Fettalkohol enthalten ist/sind,
weiterhin 0,5 - 5 Gew.-%, bezogen auf den gesamten Stift, mindestens einer Fettkomponente mit einem Schmelzpunkt von 75 - 120 °C und 0,5 - 8 Gew.-%, bezogen auf den gesamten Stift, mindestens einer Fettkomponente mit einem Schmelzpunkt von 25 - 45 °C,
weiterhin 0,5 - 30 Gew.-%, bevorzugt 1 - 25 Gew.-%, besonders bevorzugt 5 - 20 Gew.-%, außerordentlich bevorzugt 10 - 15 Gew.-%, mindestens eines Füllstoffes, der besonders bevorzugt ausgewählt ist aus Talkum, Cellulosepulvern, Stärken und Stärkederivaten.

Alle Mengenangaben beziehen sich, sofern nicht anders angegeben, auf das Gewicht der erfindungsgemäßen Zusammensetzung.

In einer besonders bevorzugten erfindungsgemäßen Ausführungsform liegt der wasserfreie Antitranspirant-Wachsstift als so genannter Mehrphasen-Stift, insbesondere als Zweiphasen-Stift, vor. Hierunter werden erfindungsgemäß Stifte verstanden, die beispielsweise eine erste Wachsstiftphase als Kern und mindestens eine zweite Wachsstiftphase als Ring um die erste Phase herum enthalten. Neben einer konzentrischen, ringförmigen Anordnung der einzelnen Phasen sind auch andere Anordnungen möglich, insbesondere eine Anordnung in Streifenförm. Die einzelnen Phasen können sich beispielsweise durch unterschiedliche Anfärbung, aber auch durch unterschiedliche Bestandteile voneinander unterscheiden. Entsprechende Mehrphasen-Stifte sind beispielsweise offenbart in US 6936242 und WO 00/67712 A1. Bevorzugte Herstellverfahren für derartige Stifte sind in US 6838032 offenbart.

Eine Ausgestaltung als Mehrphasen-Stift, insbesondere als Zweiphasen-Stift, bei dem nur eine der Phasen einen bestimmten Wirkstoff enthält, ist besonders bevorzugt.

Die Applikation kann z.B. auch mit Substraten erfolgen, die mit einer erfindungsgemäßen Zubereitung beaufschlagt sind. Besonders bevorzugt sind Feuchttücher, d.h. für den Anwender vorgefertigte, vorzugsweise einzeln abgepackte, Feuchttücher, wie sie z. B. aus dem Bereich der Glasreinigung (Brillenputztücher) oder aus dem Bereich der feuchten Toilettenpapiere wohlbekannt sind. Solche Feuchttücher, die bevorzugt auch Konservierungsstoffe enthalten können, sind dann mit einem erfindungsgemäßen Mittel imprägniert oder beaufschlagt. Sie können z. B. als Antitranspirant-Tuch eingesetzt werden, was besonders interessant für den Gebrauch unterwegs ist. Besonders bevorzugt kann es sein, wenn diese Tücher einzeln verpackt sind.

Bevorzugte Substratmaterialien sind ausgewählt aus flächigen Tüchern. Sie können aus einem faserigen oder zellulären flexiblen Material bestehen, das ausreichend mechanische Stabilität und gleichzeitig Weichheit zur Anwendung auf der Haut aufweist. Zu diesen Tüchern gehören Tücher aus gewebtem und ungewebtem synthetischen und natürlichen Fasern, Filz, Papier oder Schaumstoff, wie hydrophilem Polyurethanschaum.

Vorzugsweise werden hier herkömmliche Tücher aus ungewebtem Material (Vliese) verwendet. Vliese sind im allgemeinen als adhäsiv gebondete faserige Produkte definiert, die eine Matte oder geschichtete Faserstruktur aufweisen, oder solche, die Fasermatten umfassen, bei denen die Fasern zufällig oder in statistischer Anordnung verteilt sind. Die Fasern können natürlich sein, wie Cellulose, Lyocell, Wolle, Seide, Jute, Hanf, Baumwolle, Lein, Sisal oder Ramie; oder synthetisch, wie Rayon, Celluloseester, Polyvinylderivate, Polyolefine, Polyamide oder Polyester. Im allgemeinen ist jeder Faserdurchmesser bzw. -titer für die vorliegende Erfindung geeignet. Die hier eingesetzten ungewebten Stoffe neigen aufgrund der zufälligen oder statistischen Anordnung von Fasern in dem ungewebten Material, die ausgezeichnete Festigkeit in allen Richtungen verleihen, nicht zum Zerreißen oder Zerfallen. Beispiele für ungewebte Stoffe, die sich als Substrate in der vorliegenden Erfindung eignen, sind beispielsweise aus WO 98/18441 bekannt. Bevorzugte poröse und flächige Reinigungstücher bestehen aus einem oder verschiedenen Fasermaterialien, insbesondere aus Baumwolle, veredelter Baumwolle, Polyamid, Polyester oder Mischungen aus diesen. Vorzugsweise weisen die Substrate in Tuchform eine Fläche von 10 bis 5000 cm², vorzugsweise von 50 bis 2000 cm², insbesondere von 100 bis 1500 cm² und besonders bevorzugt von 200 bis 1000 cm² auf. Das Flächengewicht oder Basisgewicht des Materials beträgt dabei üblicherweise zwischen 20 und 1000 g/m², vorzugsweise von 30 bis 500 g/m² und insbesondere von 50 bis 150 g/m². Erfindungsgemäß bevorzugte deodorierende Substrate können durch Tränken oder Imprägnierung oder auch durch Aufschmelzen der erfindungsgemäßen Zusammensetzung auf ein Substrat erhalten werden.

Duft- und Riechstoffe zählen erfindungsgemäß nicht zu den kosmetischen Ölen, die bei der Berechnung des Gewichtsanteils der vorstehend beschriebenen Trägeröle b) berücksichtigt werden. Beispiele für Duft- und Riechstoffverbindungen vom Typ der Ester sind Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Beispiele für Duft- und Riechstoffverbindungen vom Typ der Ether sind Benzylethylether und Ambroxan, Beispiele für Duft- und Riechstoffverbindungen vom Typ der Aldehyde sind die linearen Alkanale mit 8 - 18 C-Atomen, Citral, Citronellal, Citronellyloxy-acetaldehyd, Cyclamenaldehyd, Lilial und Bourgeonal, Beispiele für Duft- und Riechstoffverbindungen vom Typ der Ketone sind die Jonone, alpha-Isomethylionon und Methylcedrylketon, Beispiele für Duft- und Riechstoffverbindungen vom Typ der Alkohole sind Anethol, Citronellol, Eugenol, Geraniol, Linalool, Phenylethylalkohol und Terpineol, Beispiele für Duft- und Riechstoffverbindungen vom Typ der Terpene sind Limonen und Pinen. Beispiele für Duft- und Riechstoffverbindungen sind Pine-, Citrus-, Jasmin-, Patchouly-, Rosen-, Ylang-Ylang-Öl, Muskateller-Salbeiöl, Kamillenöl, Nelkenöl, Minzöl, Zimtblätteröl, Lindenblütenöl, Wacholderbeeröl, Vetiveröl, Olibanumöl, Galbanumöl, Labdanumöl, Orangenblütenöl, Neroliöl, Orangenschalenöl und Sandelholzöl, weiterhin die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Niaouliöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Stemanisöl, Terpentinöl, Thujaöl, Thymianöl, Verbenaöl, Wacholderbeeröl, Wermutöl, Wintergrünöl, Ysop-Öl, Zimtöl, Zitronellöl, Zitronenöl und Zypressenöl. Weitere Duft- und Riechstoffverbindungen sind Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bomylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadecanolid, β-Phenylethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undecalacton, Vanillin, Veratrumaldehyd, Zimtaldehyd, Zimatalkohol, Zimtsäure, Zimtsäureethylester und Zimtsäurebenzylester. Weitere (leichter flüchtige) Riechstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral und Zitronellal.

Bevorzugt werden Mischungen verschiedener Duftstoffe verwendet, die gemeinsam eine ansprechende Duftnote erzeugen.

Geeignete Parfümöle können auch natürliche Riechstoffgemische enthalten, wie sie aus pflanzlichen oder tierischen Quellen zugänglich sind, z.B. Pinien-, Citrus-, Jasmin-, Rosen-, Lilien- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Melissenöl, Minzenöl, Zimtblätteröl, Lindenblütenöl, Wacholderbeerenöl, Vetiveröl, Olibanöl, Galbanumöl, Laudanumöl, Gewürznelkenöl, iso-Eugenol, Thymianöl, Bergamotteöl, Geraniumöl und Rosenöl.

Bevorzugte erfindungsgemäße Zusammensetzungen sind dadurch gekennzeichnet, dass mindestens ein Duftstoff in einer Gesamtmenge von 0,1 - 15 Gew.-%, bevorzugt 0,5 - 10 Gew.-%, besonders bevorzugt 1 - 8 Gew.-%, außerordentlich bevorzugt 2 - 7 Gew.-%, weiterhin außerordentlich bevorzugt 3 - 6 Gew.-%, jeweils bezogen auf das Gesamtgewicht der Zusammensetzung, enthalten ist.

Weitere erfindungsgemäß bevorzugte Zusammensetzungen sind gekennzeichnet durch einen Gehalt an mindestens einem so genannten "hautkühlende Wirkstoff". Unter hautkühlenden Wirkstoffen im Sinne der vorliegenden Anmeldung werden Wirkstoffe verstanden, die bei Applikation auf die Haut infolge Oberflächenanästhesierung und Reizung der kälteempfindlichen Nerven bei Migräne und dergleichen ein angenehmes Kältegefühl erzeugen, auch wenn die behandelten Hautpartien tatsächlich normale bzw. erhöhte Temperatur zeigen.

Bevorzugte hautkühlende Wirkstoffe sind insbesondere Menthol, Isopulegol sowie Mentholderivate, z. B. Menthyllactat, Menthylpyrrolidoncarbonsäure, Menthylmethylether, Menthoxypropandiol, Menthonglycerinacetal (9-Methyl-6-(1-methylethyl)-1,4-dioxaspiro(4.5)decan-2-methanol), Monomenthylsuccinat und 2-Hydroxymethyl-3,5,5-trimethylcyclohexanol. Als hautkühlende Wirkstoffe besonders bevorzugt sind Menthol, Isopulegol, Menthyllactat, Menthoxypropandiol und Menthylpyrrolidoncarbonsäure.

Bevorzugte erfindungsgemäße Zusammensetzungen enthalten mindestens einen hautkühlenden Wirkstoff in Gesamtmengen von 0,01 - 1 Gew.-%, bevorzugt 0,02 - 0,5 Gew.-% und besonders bevorzugt 0,05 - 0,2 Gew.-%, jeweils bezogen auf das Gesamtgewicht der Zusammensetzung. Bevorzugte erfindungsgemäße Zusammensetzungen sind dadurch gekennzeichnet, dass mindestens ein verkapselter Wirkstoff enthalten ist. Die Wirkstoffe, die vorteilhafterweise verkapselt sein können, sind insbesondere Duftstoffe, Parfümöle und/oder hautkühlende Wirkstoffe, aber auch andere hautpflegende Wirkstoffe, wie Vitamine, Antioxidantien etc.

Als Kapselmaterial bevorzugt sind wasserlösliche Polymere wie Stärke, physikalisch und/oder chemisch modifizierte Stärken, Cellulosederivate, wie z. B. Carboxymethylcellulose, Methylcellulose, Hydroxyethylcellulose oder Hydroxypropylmethylcellulose, Carragheene, Alginate, Maltodextrine, Dextrine, Pflanzengummen, Pektine, Xanthane, Polyvinylacetat und Polyvinylalkohol, Polyvinylpyrrolidin, Polyamide, Polyester und Homo- und Copolymere aus Monomeren, ausgewählt aus Acrylsäure, Methacrylsäure, Maleinsäure, Fumarsäure, Itaconsäure sowie den Estern und den Salzen dieser Säuren, sowie beliebige Mischungen dieser Polymeren. Bevorzugte Kapselmaterialien sind chemisch modifizierte Stärken, insbesondere Aluminiumstärkeoctenylsuccinat, z. B. das Handelsprodukt Dry Flo Plus von National Starch, oder Natriumstärkeoctenylsuccinat, z. B. das Handelsprodukt Capsul von National Starch, des weiteren Carboxymethylcellulose, Carboxymethylcellulose, Methylcellulose, Hydroxyethylcellulose und Hydroxypropylmethylcellulose, Ethylcellulose, z. B. das Handelsprodukt Tylose H 10 von Clariant, weiterhin Carragheene, Alginate und Maltodextrine, sowie beliebige Mischungen dieser Polymere.

In einer weiteren erfindungsgemäß bevorzugten Ausführungsform enthalten die erfindungsgemäßen Zusammensetzungen 0 bis maximal 50 Gew.-%, bevorzugt 1 bis 40 Gew.-%, besonders bevorzugt 2 - 30 Gew.-%, außerordentlich bevorzugt 5 - 15 Gew.-% Ethanol. Bei höheren Ethanolgehalten sollte wasserfreies (absolutes) Ethanol eingesetzt werden.

Weiterhin können die erfindungsgemäßen Zusammensetzungen zusätzliche Deodorant-Wirkstoffe enthalten. Als Deodorant-Wirkstoffe können antimikrobielle, antibakterielle oder keimhemmende Stoffe, Antioxidantien oder Geruchsadsorbentien (z. B. Zinkricinoleat) eingesetzt werden. Geeignete antimikrobielle, antibakterielle oder keimhemmende Stoffe sind insbesondere Organohalogenverbindungen sowie -halogenide, quartäre Ammoniumverbindungen, eine Reihe von Pflanzenextrakten und Zinkverbindungen. Bevorzugt sind halogenierte Phenolderivate wie z. B. Hexachlorophen oder Irgasan DP 300 (Triclosan, 2,4,4'-Trichlor-2'-hydroxydiphenylether), 3,4,4'-Trichlorcarbonilid, Chlorhexidin (1,1'-Hexamethylen-bis-[5-(4-chlorphenyl)]-biguanid), Chlorhexidingluconat, Benzalkoniumhalogenide und Cetylpyridiniumchlorid. Desweiteren sind Natriumbicarbonat, Natriumphenolsulfonat und Zinkphenolsulfonat sowie z. B. die Bestandteile des Lindenblütenöls einsetzbar. Auch schwächer wirksame antimikrobielle Stoffe, die aber eine spezifische Wirkung gegen die für die Schweißzersetzung verantwortlichen grampositiven Keime haben, können als Deodorant-Wirkstoffe eingesetzt werden. Auch Benzylalkohol kann als Deodorant-Wirkstoff eingesetzt werden. Weitere antibakteriell wirksame Deodorantien sind Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester sowie alkylierte Mono- und Oligosaccharide. Bevorzugte Deodorant-Wirkstoffe sind langkettige Diole, z. B. 1,2-Alkan-(C₅-C₁₈)-Diole, Glycerinmono(C₈-C₁₈)-Fettsäureester oder, besonders bevorzugt, Glycerinmono-(C₆-C₁₆)-alkylether, insbesondere 2-Ethylhexylglycerinether, die sehr gut haut- und schleimhautverträglich und gegen Corynebakterien wirksam sind, sowie weiterhin Phenoxyethanol, Phenoxyisopropanol (3-Phenoxy-propan-2-ol), Anisalkohol, 2-Methyl-5-phenyl-pentan-1-ol, 1,1-Dimethyl-3-phenyl-propan-1-ol, Benzylalkohol, 2-Phenylethan-1-ol, 3-Phenylpropan-1-ol, 4-Phenylbutan-1-ol, 5-Phenylpentan-1-ol, 2-Benzylheptan-1-ol, 2,2-Dimethyl-3-phenylpropan-1-ol, 2,2-Dimethyl-3-(3'-methylphenyl)-propan-l-ol, 2-Ethyl-3-phenylpropan-1-ol, 2-Ethyl-3-(3'-methylphenyl)-propan-l-ol, 3-(3'-Chlorphenyl)-2-ethylpropan-1-ol, 3-(2'-Chlorphenyl)-2-ethylpropan-1-ol, 3-(4'-Chiorphenyl)-2-ethylpropan-1-ol, 3-(3',4'-Dichlorphenyl)-2-ethylpropan-1-ol, 2-Ethyl-3-(2'-methylphenyl)-propan-1-ol, 2-Ethyl-3-(4'-methylphenyl)-propan-1-ol, 3-(3',4'-Dimethylphenyl)-2-ethylpropan-1-ol, 2-Ethyl-3-(4'-methoxyphenyl)-propan-1-ol, 3-(3',4'-Dimethoxyphenyl)-2-ethylpropan-1-ol, 2-Allyl-3-phenylpropan-1-ol und 2-n-Pentyl-3-phenylpropan-1-ol.

Auch komplexbildende Stoffe können die deodorierende Wirkung unterstützen, indem sie die oxidativ katalytisch wirkenden Schwermetallionen (z. B. Eisen oder Kupfer) stabil komplexieren. Bevorzugte Komplexbildner sind z. B. die Salze der Ethylendiamintetraessigsäure oder der Nitrilotriessigsäure sowie die Salze der 1-Hydroxyethan-1,1-diphosphonsäure.

Die Zusammensetzung einiger bevorzugter erfindungsgemäßer Zusammensetzungen können den folgenden Tabellen entnommen werden (alle Angaben in Gew.-%, bezogen auf das Gesamtgewicht der Zusammensetzung):

| | 1 | 2 | 3 | 4 |
|---|---|---|---|---|
| Schweißhemmender Wirkstoff (USP) | 5,0 bis 40 | 10 bis 35 | 15 bis 28 | 23 bis 27 |
| flüssiges Öl als Träger | 30 bis 95 | 40 bis 60 | 45 bis 55 | 40 bis 55 |
| freies Wasser | 0 bis 7 | 0 bis 7 | 0 bis 3 | 0 bis 3 |
| ACTIVATOR-(I)* | 0,01 bis 5 | 0,1 bis 3 | 0,5 bis 2 | 1 bis 1,5 |

| | | | | |
|---|---|---|---|---|
| * worin R¹ ein aliphatischer Kohlenwasserstoffrest mit 1 bis 3 C-Atomen, R² ein aliphatischer Kohlenwasserstoffrest mit 8 bis 30 C-Atomen, m eine rationale Zahl von 10 bis 50, n eine rationale Zahl von 0 bis 10, p eine rationale Zahl von 1 bis 10 | | | | |

ist.

| | 5 | 6 | 7 | 8 |
|---|---|---|---|---|
| Schweißhemmendes Aluminiumsalz (USP) | 5,0 bis 40 | 10 bis 35 | 15 bis 28 | 23 bis 27 |
| flüssiges Öl als Träger | 30 bis 95 | 40 bis 60 | 45 bis 55 | 40 bis 55 |
| freies Wasser | 0 bis 7 | 0 bis 7 | 0 bis 3 | 0 bis 3 |
| ACTIVATOR-(I)* | 0,01 bis 5 | 0,1 bis 3 | 0,5 bis 2 | 1 bis 1,5 |

| | | | | |
|---|---|---|---|---|
| * worin R¹ ein aliphatischer Kohlenwasserstoffrest mit 1 bis 3 C-Atomen, R² ein aliphatischer Kohlenwasserstoffrest mit 8 bis 30 C-Atomen, m eine rationale Zahl von 10 bis 50, n eine rationale Zahl von 0 bis 10, p eine rationale Zahl von 1 bis 10 | | | | |

ist.

| | 9 | 10 | 11 | 12 |
|---|---|---|---|---|
| Schweißhemmendes Aluminiumsalz (USP) | 5,0 bis 40 | 10 bis 35 | 15 bis 28 | 23 bis 27 |
| flüssiges Öl als Träger | 30 bis 95 | 40 bis 60 | 45 bis 55 | 40 bis 55 |
| freies Wasser | 0 bis 7 | 0 bis 7 | 0 bis 3 | 0 bis 3 |
| ACTIVATOR-(I) ** | 0,01 bis 5 | 0,1 bis 3 | 0,5 bis 2 | 1 bis 1,5 |

| | | | | |
|---|---|---|---|---|
| ** worin R¹ eine Methylgruppe, R² eine n-Decylgruppe, m eine rationale Zahl von 21 bis 23, n = 0, p eine rationale Zahl von 4 bis 8 | | | | |

ist.

| | 13 | 14 | 15 | 16 |
|---|---|---|---|---|
| Schweißhemmendes Aluminiumsalz (USP) | 5,0 bis 40 | 10 bis 35 | 15 bis 28 | 23 bis 27 |
| flüssiges Öl als Träger | 30 bis 95 | 40 bis 60 | 45 bis 55 | 40 bis 55 |
| freies Wasser | 0 bis 7 | 0 bis 7 | 0 bis 3 | 0 bis 3 |
| Methoxy PEG-22/Dodecyl Glycol Copolymer | 0,01 bis 5 | 0,1 bis 3 | 0,5 bis 2 | 1 bis 1,5 |
| | | | | |

| | 17 | 18 | 19 | 20 |
|---|---|---|---|---|
| Schweißhemmendes Aluminiumsalz (USP) | 5,0 bis 40 | 10 bis 35 | 15 bis 28 | 23 bis 27 |
| flüchtige cyclische Siliconöle (Cyclomethicone) | 30 bis 95 | 40 bis 60 | 45 bis 55 | 40 bis 55 |
| freies Wasser | 0 bis 7 | 0 bis 7 | 0 bis 3 | 0 bis 3 |
| ACTIVATOR-(I)** | 0,01 bis 5 | 0,1 bis 3 | 0,5 bis 2 | 1 bis 1,5 |

| | | | | |
|---|---|---|---|---|
| ** worin R¹ eine Methylgruppe, R² eine n-Decylgruppe, m eine rationale Zahl von 21 bis 23, n = 0, p eine rationale Zahl von 4 bis 8 | | | | |

ist.

| | 21 | 22 | 23 | 24 |
|---|---|---|---|---|
| Schweißhemmendes Aluminiumsalz (USP) | 5,0 bis 40 | 10 bis 35 | 15 bis 28 | 23 bis 27 |
| flüchtige cyclische Siliconöle (Cyclomethicone) | 30 bis 95 | 40 bis 60 | 45 bis 55 | 40 bis 55 |
| freies Wasser | 0 bis 7 | 0 bis 7 | 0 bis 3 | 0 bis 3 |
| Methoxy PEG-22/Dodecyl Glycol Copolymer | 0,01 bis 5 | 0,1 bis 3 | 0,5 bis 2 | 1 bis 1,5 |
| | | | | |

| | 25 | 26 | 27 | 28 |
|---|---|---|---|---|
| Schweißhemmendes Aluminiumsalz (USP) | 5,0 bis 40 | 10 bis 35 | 15 bis 28 | 23 bis 27 |
| flüchtige lineare Polydimethylsiloxane mit 2 bis 10 Siloxaneinheiten | 30 bis 95 | 40 bis 60 | 45 bis 55 | 40 bis 55 |
| freies Wasser | 0 bis 7 | 0 bis 7 | 0 bis 3 | 0 bis 3 |
| ACTIVATOR-(I)** | 0,01 bis 5 | 0,1 bis 3 | 0,5 bis 2 | 1 bis 1,5 |

| | | | | |
|---|---|---|---|---|
| ** worin R¹ eine Methylgruppe, R² eine n-Decylgruppe, m eine rationale Zahl von 21 bis 23, n = 0, p eine rationale Zahl von 4 bis 8 | | | | |

ist.

| | 29 | 30 | 31 | 32 |
|---|---|---|---|---|
| Schweißhemmendes Aluminiumsalz (USP) | 5,0 bis 40 | 10 bis 35 | 15 bis 28 | 23 bis 27 |
| flüchtige lineare Polydimethylsiloxane mit 2 bis 10 Siloxaneinheiten | 30 bis 95 | 40 bis 60 | 45 bis 55 | 40 bis 55 |
| freies Wasser | 0 bis 7 | 0 bis 7 | 0 bis 3 | 0 bis 3 |
| Methoxy PEG-22/Dodecyl Glycol Copolymer | 0,01 bis 5 | 0,1 bis 3 | 0,5 bis 2 | 1 bis 1,5 |
| | | | | |

| | 33 | 34 | 35 | 36 |
|---|---|---|---|---|
| Schweißhemmendes Aluminiumsalz (USP) | 5,0 bis 40 | 10 bis 35 | 15 bis 28 | 23 bis 27 |
| i) Esteröl, ausgewählt aus 2-Ethylhexylpalmitat, Isopropylmyristat und Isopropylpalmitat, und ii) mindestens ein Isoparaffinöl, ausgewählt aus Isodecan, Isoundecan, Isododecan, Isotridecan, Isotetradecan, Isopentadecan, Isohexadecan und Isoeicosan, und iii) 0 - maximal 1 Gew.-% Cyclomethicone | 30 bis 95 | 40 bis 60 | 45 bis 55 | 40 bis 55 |
| freies Wasser | 0 bis 7 | 0 bis 7 | 0 bis 3 | 0 bis 3 |
| ACTIVATOR-(I)** | 0,01 bis 5 | 0,1 bis 3 | 0,5 bis 2 | 1 bis 1,5 |

| | | | | |
|---|---|---|---|---|
| ** worin R¹ eine Methylgruppe, R² eine n-Decylgruppe, m eine rationale Zahl von 21 bis 23, n = 0, p eine rationale Zahl von 4 bis 8 | | | | |

ist.

| | 37 | 38 | 39 | 40 |
|---|---|---|---|---|
| Schweißhemmendes Aluminiumsalz (USP) | 5,0 bis 40 | 10 bis 35 | 15 bis 28 | 23 bis 27 |
| i) Esteröl, ausgewählt aus 2-Ethylhexyl-palmitat, Isopropylmyristat und Isopropylpalmitat, und ii) mindestens ein Isoparaffinöl, ausgewählt aus Isodecan, Isoundecan, Isododecan, Isotridecan, Isotetradecan, Isopentadecan, Isohexadecan und Isoeicosan, und iii) 0 - maximal 1 Gew.-% Cyclomethicone | 30 bis 95 | 40 bis 60 | 45 bis 55 | 40 bis 55 |
| freies Wasser | 0 bis 7 | 0 bis 7 | 0 bis 3 | 0 bis 3 |
| Methoxy PEG-22/Dodecyl Glycol Copolymer | 0,01 bis 5 | 0,1 bis 3 | 0,5 bis 2 | 1 bis 1,5 |
| | | | | |

| | 41 | 42 | 43 | 44 |
|---|---|---|---|---|
| Schweißhemmendes Aluminiumsalz (USP) | 5,0 bis 40 | 10 bis 35 | 15 bis 28 | 23 bis 27 |
| Triethylcitrat/Ester/C₁₀-C₁₃-Isoparaffin | 30 bis 95 | 40 bis 60 | 45 bis 55 | 40 bis 55 |
| freies Wasser | 0 bis 7 | 0 bis 7 | 0 bis 3 | 0 bis 3 |
| ACTIVATOR-(I)** | 0,01 bis 5 | 0,1 bis 3 | 0,5 bis 2 | 1 bis 1,5 |

| | | | | |
|---|---|---|---|---|
| ** worin R¹ eine Methylgruppe, R² eine n-Decylgruppe, m eine rationale Zahl von 21 bis 23, n = 0, p eine rationale Zahl von 4 bis 8 | | | | |

ist.

| | 45 | 46 | 47 | 48 |
|---|---|---|---|---|
| Schweißhemmendes Aluminiumsalz (USP) | 5,0 bis 40 | 10 bis 35 | 15 bis 28 | 23 bis 27 |
| Triethylcitrat/Ester/C₁₀-C₁₃-Isoparaffin | 30 bis 95 | 40 bis 60 | 45 bis 55 | 40 bis 55 |
| freies Wasser | 0 bis 7 | 0 bis 7 | 0 bis 3 | 0 bis 3 |
| Methoxy PEG-22/Dodecyl Glycol Copolymer | 0,01 bis 5 | 0,1 bis 3 | 0,5 bis 2 | 1 bis 1,5 |
| | | | | |

| | 49 | 50 | 51 | 52 |
|---|---|---|---|---|
| Schweißhemmendes Aluminiumsalz (USP) | 5,0 bis 40 | 10 bis 35 | 15 bis 28 | 23 bis 27 |
| Triethylcitrat/Ester/C₁₀-C₁₃-Isoparaffin | 30 bis 95 | 40 bis 60 | 45 bis 55 | 40 bis 55 |
| freies Wasser | 0 bis 7 | 0 bis 7 | 0 bis 3 | 0 bis 3 |
| ACTIVATOR-(I)** | 0,01 bis 5 | 0,1 bis 3 | 0,5 bis 2 | 1 bis 1,5 |
| Cyclomethicone | 0 bis 2 | 0 bis 2 | 0 bis 1 | 0 bis 1 |

| | | | | |
|---|---|---|---|---|
| ** worin R¹ eine Methylgruppe, R² eine n-Decylgruppe, m eine rationale Zahl von 21 bis 23, n = 0, p eine rationale Zahl von 4 bis 8 | | | | |

ist.

| | 53 | 54 | 55 | 56 |
|---|---|---|---|---|
| Schweißhemmendes Aluminiumsalz (USP) | 5,0 bis 40 | 10 bis 35 | 15 bis 28 | 23 bis 27 |
| Triethylcitrat/Ester/C₁₀-C₁₃-Isoparaffin | 30 bis 95 | 40 bis 60 | 45 bis 55 | 40 bis 55 |
| freies Wasser | 0 bis 7 | 0 bis 7 | 0 bis 3 | 0 bis 3 |
| Methoxy PEG-22/Dodecyl Glycol Copolymer | 0,01 bis 5 | 0,1 bis 3 | 0,5 bis 2 | 1 bis 1,5 |
| Cyclomethicone | 0 bis 2 | 0 bis 2 | 0 bis 1 | 0 bis 1 |

Ein weiterer Gegenstand der vorliegenden Anmeldung ist die Verwendung mindestens eines Alkyl-modifizierten Polyethers der allgemeinen Formel ACTIVATOR-(I) 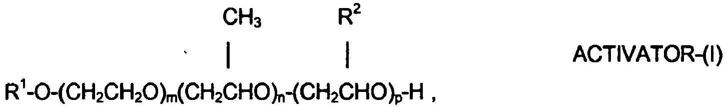 worin R¹ ein aliphatischer Kohlenwasserstoffrest mit 1 bis 3 C-Atomen, R² ein aliphatischer Kohlenwasserstoffrest mit 8 bis 30 C-Atomen, m eine rationale Zahl von 10 bis 50, n eine rationale Zahl von 0 bis 10 und p eine rationale Zahl von 1 bis 10 bedeutet, in einer schweißhemmenden Zusammensetzung, enthaltend mindestens einen schweißhemmenden Wirkstoff und 0 - 7 Gew.-%, bevorzugt 0 - 3 Gew.-%, freies Wasser, zur Verbesserung der Schweißreduktion.

Unter "Verbesserung der Schweißreduktion" ist erfindungsgemäß sowohl eine Reduzierung der Schweißmenge als auch eine Beschleunigung der Freisetzung des schweißhemmenden Wirkstoffs aus der erfindungsgemäßen Zusammensetzung zu verstehen.

Ein weiterer Gegenstand der vorliegenden Anmeldung ist die Verwendung mindestens eines Alkyl-modifizierten Polyethers der allgemeinen Formel ACTIVATOR-(I) 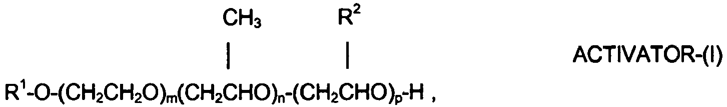 worin R¹ ein aliphatischer Kohlenwasserstoffrest mit 1 bis 3 C-Atomen, R² ein aliphatischer Kohlenwasserstoffrest mit 8 bis 30 C-Atomen, m eine rationale Zahl von 10 bis 50, n eine rationale Zahl von 0 bis 10 und p eine rationale Zahl von 1 bis 10 bedeutet, in einer schweißhemmenden Zusammensetzung, enthaltend mindestens einen schweißhemmenden Wirkstoff und 0 - 7 Gew.-%, bevorzugt 0 - 3 Gew.-%, freies Wasser, zur Verbesserung der Schweißreduktion, wobei der Alkyl-modifizierte Polyether in einer Zusammensetzung gemäß einem der Ansprüche 1 - 12 vorliegt.

Ein weiterer Gegenstand der vorliegenden Anmeldung ist die nicht-therapeutische, kosmetische Verwendung einer schweißhemmenden Zusammensetzung gemäß Anspruch 1, 2, 3, 4, 5, 6, 7, 8, 9, 10, 11 oder 12 zur Reduzierung und/oder Regulierung der Transpiration und/oder des Körpergeruchs.

Bezüglich weiterer bevorzugter Ausführungsformen der erfindungsgemäßen Verwendungen gilt mutatis mutandis das zu den erfindungsgemäßen Zusammensetzungen Gesagte.

Ein weiterer Gegenstand der vorliegenden Anmeldung ist ein nicht-therapeutisches, kosmetisches Verfahren zur Reduzierung und/oder Regulierung der Schweißbildung und/oder des Körpergeruchs, bei dem eine erfindungsgemäße oder erfindungsgemäß bevorzugte Zusammensetzung gemäß einem der Ansprüche 1, 2, 3, 4, 5, 6, 7, 8, 9, 10, 11 oder 12 in einer wirksamen Menge auf die Haut, bevorzugt auf die Haut im Achselbereich, aufgetragen wird.

Bezüglich weiterer bevorzugter Ausführungsformen des erfindungsgemäßen Verfahrens gilt mutatis mutandis das zu den erfindungsgemäßen Zusammensetzungen Gesagte.

Die nachfolgenden Beispiele sollen die Erfindung verdeutlichen, ohne sie hierauf zu beschränken. Schweißhemmende Suspensions-Zusammensetzungen zur Konfektionierung als Roll-on in einem Rollkugel-Spender (alle Mengenangaben sind in Gew.-%).

| Handelsname | INCI | Nr. 1.1 | Nr. 1.2 |
|---|---|---|---|
| | | | |
| | Activated Aluminium Zirconium Pentachlorohydrex - Gly | 22,0 | - |
| Reach AZP 908 | Activated Aluminium Zirconium Tetrachlorohydrex - Gly | - | 24 |
| | Isopropyl myristate | 70,6 | 64,8 |
| Elfacos E 200 | Methoxy PEG-22/Dodecyl Glycol Copolymer | 2 | 2 |
| Propylencarbonat | Propylene Carbonate | 0,9 | 1,2 |
| Bentone 38 V CG | Disteardimonium Hectorite | 3,8 | 5 |
| Parfum InCaps | Incapsulated Fragrance | 0,2 | - |
| Parfum | Fragrance | 0,5 | 1 |

**Tabelle 2: Antitranspirant-Wachsstifte**

| | 2.1 | 2.2 | 2.3 | 2.4 | 2.5 | 2.6 | 2.7 |
|---|---|---|---|---|---|---|---|
| | | | | | | | |
| PPG-14 Butylether | 15,00 | 18,00 | 12,00 | 19,00 | 6,00 | 10,50 | 10,50 |
| Hydrogenated Castor Oil | 1,00 | 1,50 | 2,00 | 1,50 | 1,80 | 3,00 | 3,00 |
| Stearyl alcohol | 20,00 | 18,00 | 15,00 | 18,00 | 17,00 | 23, 50 | 23,50 |
| Ceteareth-30 | 3,00 | 2,00 | 4,00 | - | 3,00 | 3,00 | - |
| Isoceteth-20 | - | - | - | 2,50 | - | - | -- |
| Elfacos E 200 | 2,0 | 1,5 | 1,5 | 1,00 | 2,0 | 1,0 | 1,0 |
| Parfum | 1,00 | 1,20 | 0,80 | 1,50 | 1,00 | 1,50 | 1,50 |
| Aluminumchlorohydrate | 20,00 | 22,00 | 18,00 | - | 20,00 | -- | -- |
| Aluminum Zirconium tetrachlorohydrate Gly | -- | -- | -- | 22,00 | -- | 20,00 | -- |
| Aluminum Zirconium pentachlorohydrate Gly | -- | -- | -- | -- | -- | -- | 20,00 |
| Allantoin | 0,10 | -- | -- | 0,10 | -- | -- | -- |
| Cocoglycerides (FP 30 - 32 °C) | 4,00 | 6,00 | 3,00 | 5,00 | 6,00 | -- | -- |
| Myristylmyristat | - | - | - | - | - | 1,70 | 1,40 |
| Silica | - | - | - | - | - | 0,80 | -- |
| Silica Dimethyl Silylate | - | - | - | - | - | 1,00 | -- |
| Talc | 3,00 | 2,00 | 5,00 | 3,00 | 3,00 | -- | -- |
| Tocopherylacetat | 0,20 | -- | 0,50 | 0,10 | -- | -- | -- |
| Cyclopentasiloxan | ad 100 | ad 100 | ad 100 | ad 100 | ad 100 | ad 100 | ad 100 |

Die erfindungsgemäßen Zusammensetzungen Nr. 1.1 - 2.7 wurden auf die Haut im Achselbereich aufgetragen.

**Tabelle 3: Antitranspirant-Wachsstifte**

| | 3.1 | 3.2 | 3.3 | 3.4 | 3.5 |
|---|---|---|---|---|---|
| | | | | | |
| Hexyldecanol | 10,00 | 12,00 | 10,00 | 8,00 | 8,00 |
| PPG-14 Butylether | 6,00 | 5,00 | 6,00 | 8,00 | 8,00 |
| Hydrogenated Castor Oil | 4,00 | 5,00 | 6,00 | 5,00 | 5,00 |
| Stearyl alcohol | 12,00 | 14,00 | 11,00 | 16,00 | 16,00 |
| Cetyl alcohol | 6,00 | 5,00 | 6,00 | 3,00 | 3,00 |
| PEG-20 Glyceryl stearate | 5,00 | 4,00 | 6,00 | 4,00 | 4,00 |
| Ceteareth-30 | 3,00 | 1,00 | 3,00 | -- | -- |
| Parfum | 1,00 | 1,20 | 0,80 | 1,00 | 1,00 |
| Aluminumchlorohydrate | 20,00 | 20,00 | 18,00 | -- | -- |
| Aluminium Zirconium tetrachlorohydrate Glyc | -- | -- | -- | 23,00 | 23,00 |
| Talc | 8,00 | 5,00 | 8,00 | 7,00 | 7,00 |
| Elfacos E 200 | 2,0 | 1,5 | 1,5 | 1,00 | 2,0 |
| Cyclopentasiloxane | ad 100 | ad 100 | ad 100 | ad 100 | ad 100 |

**Tabelle 4: Wasserfreie Antitranspirant-Wachsstifte ohne Cyclomethicone**

| | 4.1 | 4.2 | 4.3 | 4.4 | 4.5 | 4.6 |
|---|---|---|---|---|---|---|
| | | | | | | |
| Stearyl Alcohol | 16 | 18 | 18 | 18 | 18 | 18 |
| Behenyl Alcohol | -- | -- | 0,2 | -- | -- | -- |
| Ceteareth-30 | 3 | 3 | 3 | 3 | 3 | 3 |
| PPG-14 Butyl Ether | 15 | 5 | 10 | 14,5 | 10 | 7,5 |
| Dimethicone (2 cSt) | **--** | -- | 18,4 | 23 | 22,5 | 23,5 |
| Dimethicone (5 cSt) | 31,5 | 43 | 16,9 | 11 | -- | -- |
| Elfacos E 200 | 2,0 | 1,5 | 1,5 | 1,00 | 2,0 | 1,5 |
| Hydrogenated Polydecene | -- | -- | -- | -- | 15 | 15 |
| Cocoglycerides (FP 30 - 32 °C) | 5 | 4 | 4 | 4 | 4 | 4 |
| Hydrogenated Castor Oil | 1,5 | 1,5 | 2 | 1,5 | 1,5 | 1,5 |
| Aluminum Chlorohydrate | 20 | 20 | 20 | 20 | 20 | 20 |
| Talc | 5 | 3 | 5 | 3 | 3 | 5 |
| Parfum | 1 | 1 | 1 | 1 | 1 | 1 |

Die erfindungsgemäßen Zusammensetzungen Nr. 3.1 - 4.6 wurden auf die Haut im Achselbereich aufgetragen.

**Tabelle 5: Wasserfreie Antitranspirant-Wachsstifte mit zwei Phasen**

| **Erste (äußere) Phase** | **5.1** | **5.2** | **5.3** |
|---|---|---|---|
| C₁₂₋₁₅ Alkyl Benzoate | 9.15 | 28.60 | 32.40 |
| Isopropyl myristate | - | - | 15.62 |
| Phenyl trimethicone (DC 556) | - | - | 2.60 |
| 12-Hydroxystearinsäure | 12.00 | 12.00 | 6.00 |
| N-lauroyl glutamic acid di-n-butylamide | 2.50 | 2.00 | 1.35 |
| C20-40 Pareth-10 | 3.00 | 3.00 | 3.00 |
| Elfacos E 200 | 2.00 | 1.00 | 1.50 |
| Aktiviertes Al-Zr Chlorohydrate Gly; 17.5 Gew.-% USP Aktivsubstanz | 33.50 | 33.50 | 33.50 |
| Parfum | 3.85 | 2.50 | 4.03 |
| Parfum (verkapselt) | - | 1.50 | - |
| Cyclomethicone (DC 245) | 34.00 | 16.20 | - |
| | | | |

| **Zweite Phase (innerer Streifen oder Zylinder)** | **5.4** | **5.5** | **5.6** |
|---|---|---|---|
| C₁₂₋₁₅ Alkyl Benzoate | 46.55 | 51.02 | 56.08 |
| Ilsopropyl myristate | - | - | 25.85 |
| Phenyl trimethicone (DC 556) | - | - | 4.30 |
| 12-Hydroxystearinsäure | 15.00 | 15.44 | 10.00 |
| N-lauroyl glutamic acid di-n-butylamide | 1.50 | 1.50 | 2.00 |
| Farbstoff | 0.10 | 0.10 | 0.07 |
| Parfum | 0.85 | 1.24 | 1.70 |
| Cyclomethicone (DC 245) | 36.00 | 30.70 | -- |

Es wurden wasserfreie Antitranspirant-Wachsstifte mit zwei Phasen hergestellt mit äußerer Phase 5.1 und innerer Phase 5.4, äußerer Phase 5.2 und innerer Phase 5.5 und mit äußerer Phase 5.3 und innerer Phase 5.6. Selbstverständlich sind auch andere Kombinationen aus äußerer und innerer Phase möglich.

Die erfindungsgemäßen Zusammensetzungen Nr. 5.1/5.3, Nr. 5.2/5.4 und Nr. 5.3/5.6 wurden auf die Haut im Achselbereich aufgetragen.

**Tabelle 6: Antiperspirant-Crème wasserfrei (Soft Solid)**

| | 6.1 | 6.2 | 6.3 | 6.4 |
|---|---|---|---|---|
| | | | | |
| Aluminum Chlorohydrate | 20,00 | 22,00 | 20,00 | -- |
| Aluminium Zirconium tetrachlorohydrate Gly | -- | -- | -- | 24,00 |
| Hexyldecanol | 5,00 | 4,50 | 5,50 | 6,00 |
| Dicaprylylether | 3,00 | 4,00 | 3,50 | 5,00 |
| Cocoglycerides | 5,00 | 6,00 | 7,00 | 3,00 |
| C18-C36 Triglyceride | 6,00 | 5,00 | 4,00 | 3,00 |
| Ceteareth-30 | 3,00 | 2,00 | 2,50 | 4,00 |
| PEG-20 Glycerylstearate | 5,00 | 6,00 | 3,00 | 2,00 |
| Elfacos E 200 | 2,00 | 2,00 | 1,00 | 1,50 |
| Cellulose | 3,00 | 2,00 | 5,00 | 1,00 |
| Aluminum-Starch-Octenylsuccinate | 5,00 | 4,00 | 6,00 | 5,00 |
| Silica | 1,00 | 2,00 | 0,50 | -- |
| Talc | 10,00 | 5,00 | 7,00 | 12,00 |
| Allantoin | 0,10 | 0,10 | -- | -- |
| Parfum | 1,00 | 1,50 | 2,00 | 0,80 |
| Cyclopentasiloxan | ad 100 | ad 100 | ad 100 | ad 100 |

**Tabelle 7: Puder (Deodorant / Antitranspirant)**

| | 7.1 | 7.2 | 7.3 | 7.4 |
|---|---|---|---|---|
| | | | | |
| Aluminum Chlorohydrate | 20,00 | -- | -- | -- |
| Aluminium Zirconium tetrachlorohydrate Gly | -- | -- | -- | 24,00 |
| Silica | 2,00 | 2,00 | 1,00 | 1,00 |
| Triclosan | -- | 0,30 | 0,10 | -- |
| Sensiva SC 50 | -- | -- | 1,00 | -- |
| Parfum | 1,00 | 0,50 | 2,00 | 1,00 |
| Parfum (verkapselt) | -- | 1,00 | -- | 1,00 |
| Menthyllactat (verkapselt) | -- | -- | 1,00 | 1,00 |
| Elfacos E 200 | 2,00 | 2,00 | 1,00 | 1,50 |
| Talc | ad 100 | ad 100 | ad 100 | ad 100 |

Die Puderbeispiele 7.1 - 7.4 können sowohl als loser Puder als auch als gepresster Puder konfektioniert sein. Die erfindungsgemäßen Zusammensetzungen Nr. 6.1 - 7.4 wurden auf die Haut im Achselbereich aufgetragen.

**Tabelle 8: Dibenzylidensorbitol-basierte Antitranspirant-Gelstifte**

| | 8.1 | 8.2 | 8.3 | 8.4 (Deodorant) |
|---|---|---|---|---|
| Al/Zr tetrachloro-hydrate-gly | 11 | 11 | 11 | 3 |
| Dibenzylidensorbitol | 1 | 1,3 | 1,1 | 1,3 |
| Hydroxypropyl-cellulose | 0,3 | 0,3 | 0,35 | 0,5 |
| Na₄EDTA | 0,2 | 0,2 | 0,2 | 0,2 |
| Diisopropylsebacat | - | 1 | 1 | - |
| Glycereth-7-diisononanoat | 0,5 | - | - | - |
| Oleth-10 | - | - | - | 0,75 |
| PPG-10-butandiol | - | - | - | 0,75 |
| PPG-3-myristylether | - | - | - | 0,75 |
| Elfacos E 200 | 1,00 | 1,00 | 1,00 | 1,00 |
| Parfüm | 1,25 | 1,25 | 1,25 | 1,25 |
| 1,2-Propylenglycol | ad 100 | ad 100 | ad 100 | ad 100 |

**Tabelle 9: Wasserfreie Roll-on-Zusammensetzungen**

| | 9.1 | 9.2 | 9.3 | 9.4 | 9.5 |
|---|---|---|---|---|---|
| Quatemium-18 hectorite | 4,0 | 2,5 | 2,5 | 3,2 | 3,6 |
| Parfum | 0,5 | 0,5 | 0,5 | 1,0 | 1,0 |
| Parfum (verkapselt) | 0,5 | 0,5 | 0,5 | 1,0 | 1,0 |
| Al/Zr tetrachlorohydrate-gly (aktiviert) | 20 | 20 | 20 | - | - |
| Al/Zr pentachlorohydrategly (aktiviert) | - | - | - | 20 | 20 |
| Elfacos E 200 | 1,00 | 1,00 | 1,00 | 1,00 | 2,00 |
| Propylencarbonat | 1,0 | 1,0 | - | - | 1,0 |
| Aerosil 300 (Fumed Silica) | - | 0,2 | 0,4 | - | - |
| Isopropylmyristat | ad 100 | ad 100 | ad 100 | ad 100 | ad 100 |

Die erfindungsgemäßen Zusammensetzungen Nr. 8.1 - 9.5 wurden auf die Haut im Achselbereich aufgetragen.

Das Entfernen der erfindungsgemäßen Zusammensetzungen (beispielsweise durch Waschen) erfolgt im Rahmen der üblichen Hygieneroutine, beispielsweise 1, 2, 3, 4, 5, 6, 7, 8, 9, 10, 11, 12 oder 24 Stunden nach der Applikation.

## Patentansprüche

1. Schweißhemmende Zusammensetzung zur persönlichen Körperpflege, konfektioniert als Nonaerosol, Stift, Soft Solid, Creme, Gel, nicht-versprühbare Suspension, nicht-versprühbare Lösung oder auf einem Substrat imprägniert, enthaltend
a) mindestens einen schweißhemmenden Wirkstoff,
b) mindestens ein unter Normalbedingungen flüssiges Öl als Träger,
c) 0 - 7 Gew.-%, bevorzugt 0 - 3 Gew.-%, freies Wasser, bezogen auf das Gewicht der Zusammensetzung,
d) mindestens einen Alkyl-modifizierten Polyether der allgemeinen Formel ACTIVATOR-(I) 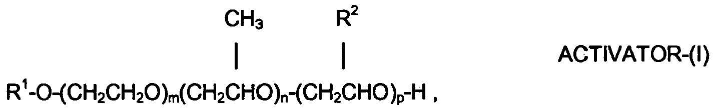
worin R¹ ein aliphatischer Kohlenwasserstoffrest mit 1 bis 3 C-Atomen,
R² ein aliphatischer Kohlenwasserstoffrest mit 8 bis 30 C-Atomen,
m eine rationale Zahl von 10 bis 50,
n eine rationale Zahl von 0 bis 10,
p eine rationale Zahl von 1 bis 10
bedeutet.

2. Zusammensetzung gemäß Anspruch 1, **dadurch gekennzeichnet, dass** der Alkyl-modifizierte Polyether d) ausgewählt ist aus Verbindungen der allgemeinen Formel ACTIVATOR-(I), worin R¹ ausgewählt ist aus einer Methylgruppe, einer Ethylgruppe, einer n-Propylgruppe und einer 1-Methylethylgruppe, bevorzugt einer Methylgruppe,
R² ausgewählt ist aus einer n-Octyl-, 2-Ethylhexyl-, n-Nonyl-, n-Decyl-, 2-Ethyloctyl-, n-Undecyl-, n-Dodecyl-, 2-Ethyldecyl-, n-Tridecyl-, Myristyl-, n-Pentadecyl-, Cetyl-, Palmityl-, Stearyl-, Elaidyl-, Arachidyl-, Behenyl- oder Cocyl-Gruppe, bevorzugt einer n-Decyl-Gruppe, m eine Zahl von 12 - 30, bevorzugt 22, darstellt,
n eine rationale Zahl von 0 - 8, bevorzugt 0 - 4, besonders bevorzugt 0, darstellt,
p eine rationale Zahl von 1 - 9, bevorzugt 4 - 8, besonders bevorzugt 5 - 7, darstellt.

3. Zusammensetzung gemäß Anspruch 1 oder 2, **dadurch gekennzeichnet, dass** der Alkylmodifizierte Polyether d) der allgemeinen Formel ACTIVATOR-(I) einen HLB-Wert im Bereich von 5 - 7, bevorzugt 6 - 6,8, besonders bevorzugt 6,2 - 6,5, aufweist.

4. Zusammensetzung gemäß einem der vorstehenden Ansprüche, **dadurch gekennzeichnet, dass** mindestens ein Alkyl-modifizierter Polyether der allgemeinen Formel ACTIVATOR-(I) enthalten ist mit einem HLB-Wert im Bereich von 5 - 7, bevorzugt 6 - 6,8, besonders bevorzugt 6,2 - 6,5, und mit R¹ = Methyl, R² = n-Decyl, m = 22, n = 0 und p = 4 - 5.

5. Zusammensetzung gemäß Anspruch 1, 2, 3 oder 4, **dadurch gekennzeichnet, dass** mindestens ein Alkyl-modifizierter Polyether der allgemeinen Formel ACTIVATOR-(I) enthalten ist mit einem HLB-Wert im Bereich von 5 - 7, bevorzugt 6 - 6,8, besonders bevorzugt 6,2 - 6,5, und mit R¹ = Methyl, R² = n-Decyl, m = 22, n = 0 und p = 7 - 8.

6. Zusammensetzung gemäß Anspruch 1, 2, 3, 4 oder 5, **dadurch gekennzeichnet, dass** der Alkyl-modifizierte Polyether d) der allgemeinen Formel ACTIVATOR-(I) ausgewählt ist aus Verbindungen mit der INCI-Bezeichnung Methoxy PEG-22/Dodecyl Glycol Copolymer.

7. Zusammensetzung gemäß einem der vorstehenden Ansprüche, **dadurch gekennzeichnet, dass** das mindestens eine unter Normalbedingungen flüssige Träger-Öl b) ausgewählt ist aus flüchtigen cyclischen oder linearen Siliconölen, nichtflüchtigen höhermolekularen linearen Dimethylpolysiloxanen, Estern von linearen oder verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, die hydroxyliert sein können, Benzoesäureestem von linearen oder verzweigten C₈₋₂₂-Alkanolen, den C₈-C₂₂-Fettalkoholestern einwertiger oder mehrwertiger C₂-C₇-Hydroxycarbonsäuren, den Anlagerungsprodukten von Ethylenoxid und/oder Propylenoxid an ein- oder mehrwertige C₃₋₂₀-Alkanole, flüssigen Paraffinölen, Isoparaffinölen, insbesondere Isodecan, Isoundecan, Isododecan, Isotridecan, Isotetradecan, Isopentadecan, Isohexadecan und Isoeicosan, synthetischen Kohlenwasserstoffen, wie Polyisobuten oder Polydecene, und alicyclischen Kohlenwasserstoffen, verzweigten gesättigten oder ungesättigten Fettalkoholen mit 6 - 30 Kohlenstoffatomen, Mischungen aus Guerbetalkoholen und Guerbetalkoholestem, den symmetrischen, unsymmetrischen oder cyclischen Estern der Kohlensäure mit Fettalkoholen, Triglyceriden von linearen oder verzweigten, gesättigten oder ungesättigten, gegebenenfalls hydroxylierten C₈₋₃₀-Fettsäuren, Dicarbonsäureestern von linearen oder verzweigten C₂-C₁₀-Alkanolen, Di-n-alkylethern mit insgesamt 12 bis 36, insbesondere 12 bis 24 C-Atomen, sowie Mischungen der vorgenannten Öle.

8. Zusammensetzung gemäß einem der vorstehenden Ansprüche, **dadurch gekennzeichnet, dass** das mindestens eine unter Normalbedingungen flüssige Träger-Öl b) mindestens ein Isoparaffinöl, insbesondere ausgewählt aus Isodecan, Isoundecan, Isododecan, Isotridecan, Isotetradecan, Isopentadecan, Isohexadecan und Isoeicosan, umfasst.

9. Zusammensetzung gemäß einem der Ansprüche 1, 2, 3, 4, 5, 6, 7 oder 8, **dadurch gekennzeichnet, dass** 0 bis maximal 1 Gew.-% Cyclomethicone enthalten ist.

10. Zusammensetzung gemäß einem der Ansprüche 1, 2, 3, 4, 5, 6, 7, 8 oder 9, **dadurch gekennzeichnet, dass** das unter Normalbedingungen flüssige Träger-Öl b) aus einer Mischung aus b)i) einem Esteröl, ausgewählt aus 2-Ethylhexylpalmitat, Isopropylmyristat und Isopropylpalmitat, und b)ii) mindestens einem Isoparaffinöl, ausgewählt aus Isodecan, Isoundecan, Isododecan, Isotridecan, Isotetradecan, Isopentadecan, Isohexadecan und Isoeicosan, und b)iii) 0 - maximal 1 Gew.-% Cyclomethicone besteht.

11. Nicht-therapeutisches, kosmetisches Verfahren zur Reduzierung und/oder Regulierung der Schweißbildung und/oder des Körpergeruchs, bei dem eine erfindungsgemäße oder erfindungsgemäß bevorzugte Zusammensetzung gemäß einem der Ansprüche 1, 2, 3, 4, 5, 6, 7, 8, 9 oder 10 in einer wirksamen Menge auf die Haut, bevorzugt auf die Haut im Achselbereich, aufgetragen wird.

12. Nicht-therapeutische, kosmetische Verwendung einer schweißhemmenden Zusammensetzung gemäß Anspruch 1, 2, 3, 4, 5, 6, 7, 8, 9 oder 10 zur Reduzierung und/oder Regulierung der Transpiration und/oder des Körpergeruchs.

13. Verwendung mindestens eines Alkyl-modifizierten Polyethers der allgemeinen Formel ACTIVATOR-(I) 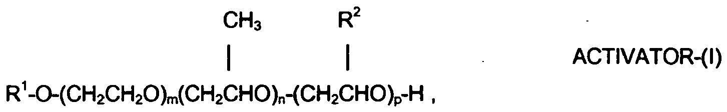
worin R¹ ein aliphatischer Kohlenwasserstoffrest mit 1 bis 3 C-Atomen,
R² ein aliphatischer Kohlenwasserstoffrest mit 8 bis 30 C-Atomen,
m eine rationale Zahl von 10 bis 50,
n eine rationale Zahl von 0 bis 10,
p eine rationale Zahl von 1 bis 10
bedeutet, in einer schweißhemmenden Zusammensetzung, enthaltend mindestens einen schweißhemmenden Wirkstoff und 0 - 7 Gew.-%, bevorzugt 0 - 3 Gew.-%, freies Wasser, zur Verbesserung der Schweißreduktion, **dadurch gekennzeichnet, dass** der Alkyl-modifizierte Polyether der allgemeinen Formel ACTIVATOR-(I) in einer Zusammensetzung gemäß einem der Ansprüche 1-10 vorliegt.

## Claims

1. An antiperspirant composition for personal body care, made up as a nonaerosol, stick, soft solid, cream, gel, non-sprayable suspension, non-sprayable solution or impregnated on a substrate, containing
a) at least one active antiperspirant substance,
b) at least one oil which is liquid under normal conditions as carrier,
c) 0 - 7 wt.%, preferably 0 - 3 wt.%, free water, based on the weight of the composition,
d) at least one alkyl-modified polyether of the general formula ACTIVATOR-(I) 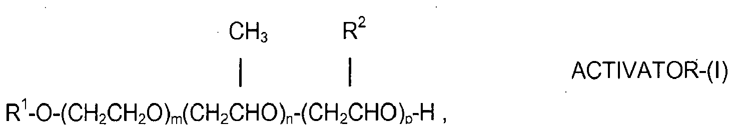
wherein R¹ signifies an aliphatic hydrocarbon residue with 1 to 3 C atoms,
R² signifies an aliphatic hydrocarbon residue with 8 to 30 C atoms,
m is a rational number from 10 to 50,
n is a rational number from 0 to 10,
p is a rational number from 1 to 10.

2. The composition according to claim 1, wherein the alkyl-modified polyether d) is selected from compounds of the general formula ACTIVATOR-(I), wherein
R¹ is selected from a methyl group, an ethyl group, an n-propyl group and a 1-methylethyl group, preferably a methyl group,
R² is selected from an n-octyl, 2-ethylhexyl, n-nonyl, n-decyl, 2-ethyloctyl, n-undecyl, n-dodecyl, 2-ethyldecyl, n-tridecyl, myristyl, n-pentadecyl, cetyl, palmityl, stearyl, elaidyl, arachidyl, behenyl or cocyl group, preferably an n-decyl group,
m represents a number from 12 - 30, preferably 22,
n represents a rational number from 0 - 8, preferably 0 - 4, particularly preferably 0,
p represents a rational number from 1 - 9, preferably 4 - 8, particularly preferably 5 - 7.

3. The composition according to claim 1 or 2, wherein the alkyl-modified polyether d) of the general formula ACTIVATOR-(I) has an HLB value in the range of 5 - 7, preferably 6 - 6.8, particularly preferably 6.2 - 6.5.

4. The composition according to one of the preceding claims, wherein at least one alkyl-modified polyether of the general formula ACTIVATOR-(I) is contained with an HLB value in the range of 5 - 7, preferably 6 - 6.8, particularly preferably 6.2 - 6.5, and with R¹ = methyl, R² = n-decyl, m = 22, n = 0 and p = 4 - 5.

5. The composition according to claim 1, 2, 3 or 4, wherein at least one alkyl-modified polyether of the general formula ACTIVATOR-(I) is contained with an HLB value in the range of 5 - 7, preferably 6 - 6.8, particularly preferably 6.2 - 6.5, and with R¹ = methyl, R² = n-decyl, m = 22, n = 0 and p = 7 - 8.

6. The composition according to claim 1, 2, 3, 4 or 5, wherein the alkyl-modified polyether d) of the general formula ACTIVATOR-(I) is selected from compounds with the INCI name Methoxy PEG-22/Dodecyl Glycol Copolymer.

7. The composition according to one of the preceding claims, wherein the at least one carrier oil b) which is liquid under normal conditions is selected from volatile cyclic or linear silicone oils, non-volatile higher molecular-weight linear dimethyl polysiloxanes, esters of linear or branched saturated or unsaturated fatty alcohols having 2 - 30 carbon atoms with linear or branched saturated or unsaturated fatty acids having 2 - 30 carbon atoms, which may be hydroxylated, benzoic acid esters of linear or branched C₈₋₂₂ alkanols, C₈-C₂₂ fatty alcohol esters of monohydric or polyhydric C₂-C₇ hydroxycarboxylic acids, the addition products of ethylene oxide and/or propylene oxide to monohydric or polyhydric C₃₋₂₀ alkanols, liquid paraffin oils, isoparaffin oils, in particular isodecane, isoundecane, isododecane, isotridecane, isotetradecane, isopentadecane, isohexadecane and isoeicosane, synthetic hydrocarbons, such as polyisobutene or polydecene, and alicyclic hydrocarbons, branched saturated or unsaturated fatty alcohols with 6 - 30 carbon atoms, mixtures of Guerbet alcohols and Guerbet alcohol esters, the symmetrical, asymmetrical or cyclic esters of carbonic acid with fatty alcohols, triglycerides of linear or branched, saturated or unsaturated, optionally hydroxylated C₈₋₃₀ fatty acids, dicarboxylic acid esters of linear or branched C₂-C₁₀ alkanols, di-n-alkyl ethers with a total of 12 to 36, in particular 12 to 24 C atoms, and mixtures of the aforementioned oils.

8. The composition according to one of the preceding claims, wherein the at least one carrier oil b) which is liquid under normal conditions comprises at least one isoparaffin oil, in particular selected from isodecane, isoundecane, isododecane, isotridecane, isotetradecane, isopentadecane, isohexadecane and isoeicosane.

9. The composition according to one of claims 1, 2, 3, 4, 5, 6, 7 or 8, wherein 0 to no more than 1 wt.% of cyclomethicones is contained.

10. The composition according to one of claims 1, 2, 3, 4, 5, 6, 7, 8 or 9, wherein the carrier oil b) which is liquid under normal conditions consists of a mixture of b)i) an ester oil, selected from 2-ethylhexyl palmitate, isopropyl myristate and isopropyl palmitate, and b)ii) at least one isoparaffin oil, selected from isodecane, isoundecane, isododecane, isotridecane, isotetradecane, isopentadecane, isohexadecane and isoeicosane, and b)iii) 0 to no more than 1 wt.% of cyclomethicones.

11. A non-therapeutic, cosmetic method of reducing and/or regulating sweat formation and/or body odor, in which a composition according to the invention, or which is preferred according to the invention, according to one of claims 1, 2, 3, 4, 5, 6, 7, 8, 9 or 10 is applied onto the skin in an effective quantity, preferably onto the skin in the arm pit area.

12. Non-therapeutic, cosmetic use of an antiperspirant composition according to claim 1, 2, 3, 4, 5, 6, 7, 8, 9 or 10 for reducing and/or regulating perspiration and/or body odor.

13. Use of at least one alkyl-modified polyether of the general formula ACTIVATOR-(I) 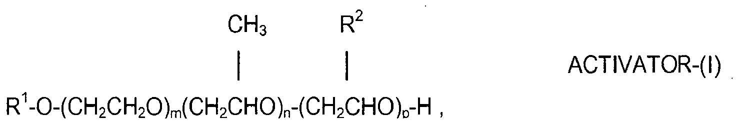
wherein R¹ signifies an aliphatic hydrocarbon residue with 1 to 3 C atoms,
R² signifies an aliphatic hydrocarbon residue with 8 to 30 C atoms,
m is a rational number from 10 to 50,
n is a rational number from 0 to 10,
p is a rational number from 1 to 10,
in an antiperspirant composition containing at least one active antiperspirant substance and 0 - 7 wt.%, preferably 0 - 3 wt.%, free water, for improving sweat reduction, wherein the alkyl-modified polyether of the general formula ACTIVATOR-(I) is present in a composition according to one of claims 1 - 10.

## Revendications

1. Composition anti-transpirante pour les soins corporels personnels, confectionnée sous forme d'un non-aérosol, d'un stylo, d'une matière solide molle, d'une crème,
d'un gel, d'une suspension non pulvérisable, d'une solution non pulvérisable ou imprégnée sur un substrat, contenant
a) au moins une substance active anti-transpirante,
b) au moins une huile servant de support qui est liquide dans des conditions normales,
c) 0 à 7% en poids, de préférence 0 à 3% en poids, d'eau libre, par rapport au poids de la composition,
d) au moins un polyéther alkylé de la formule générale ACTIVATEUR-(I) 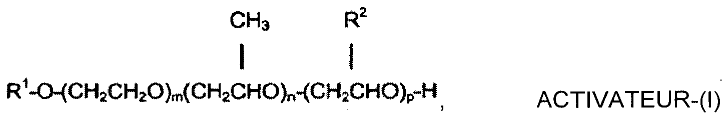 dans laquelle
R¹ est un radical hydrocarboné aliphatique ayant 1 à 3 atomes de carbone,
R² est un radical hydrocarboné aliphatique ayant 8 à 30 atomes de carbone,
m est un nombre rationnel compris entre 10 et 50,
n est un nombre rationnel compris entre 0 et 10,
p est un nombre rationnel compris 1 entre 10.

2. Composition selon la revendication 1, **caractérisée en ce que** le polyéther alkylé d) est choisi parmi les composés de la formule générale ACTIVATEUR-(I) dans laquelle
R¹ est choisi parmi un groupe méthyle, un groupe éthyle, un groupe n-propyle et un groupe 1-méthyléthyle, de préférence un groupe méthyle,
R² est choisi parmi les groupes n-octyl-, 2-éthylhexyl-, n-nonyl-, n-décyl-, 2-éthyloctyl-, n-undécyl-, n-dodécyl-, 2-éthyldécyl-, n-tridécyl-, myristyl-, n-pentadécyl-, cétyl-, palmityl-, stéaryl-, élaïdyl-, arachidyl-, béhényl- ou cocyl-, de préférence un groupe n-décyl-,
m est un nombre compris entre 12 et 30, de préférence 22,
n est un nombre rationnel compris entre 0 et 8, de préférence entre 0 et 4, plus préférablement 0,
p est un nombre rationnel compris entre 1 et 9, de préférence entre 4 et 8, plus préférablement entre 5 et 7.

3. Composition selon la revendication 1 ou 2, **caractérisé en ce que** le polyéther alkylé d) de la formule générale ACTIVATEUR-(I) a une valeur HLB dans la gamme de 5 à 7, de préférence de 6 à 6,8, plus préférablement de 6,2 à 6,5.

4. Composition selon l'une des revendications précédentes, **caractérisée en ce qu'**elle contient au moins un polyéther alkylé de la formule générale ACTIVATEUR-(I) ayant une valeur HLB dans la gamme de 5 à 7, de préférence de 6 à 6,8, plus préférablement de 6,2 à 6.5, et avec R¹ = méthyle, R² = n-décyle, m = 22, n = 0 et p = 4 à 5.

5. Composition selon la revendication 1, 2, 3 ou 4, **caractérisée en ce qu'**elle contient au moins un polyéther alkylé de la formule générale ACTIVATEUR-(I) ayant une valeur HLB dans la gamme de 5 à 7, de préférence de 6 à 6,8, plus préférablement de 6,2 à 6,5, et avec R¹ = méthyle, R² = n-décyle, m = 22, n = 0 et p = 7 à 8.

6. Composition selon la revendication 1, 2, 3, 4 ou 5, **caractérisée en ce que** le polyéther alkylé d) de la formule générale ACTIVATEUR-(I) est choisi parmi les composés ayant la dénomination INCI copolymère méthoxy PEG-22/Dodécyl Glycol.

7. Composition selon l'une des revendications précédentes, **caractérisée en ce que** l'au moins une huile de support b), liquide dans des conditions normales, est choisie parmi les huiles de silicone cycliques ou linéaires volatiles, les diméthylpolysiloxanes linéaires de poids moléculaire élevé non volatiles, les esters d'alcools gras linéaires ou ramifiés, saturés ou insaturés, ayant de 2 à 30 atomes de carbone, avec des acides gras linéaires ou ramifiés, saturés ou insaturés, ayant de 2 à 30 atomes de carbone, qui peuvent être hydroxylés, des esters d'acide benzoïque d'alcanols en C₈ à C₂₂ linéaires ou ramifiés, des esters d'alcools gras en C₈ à C₂₂ d'acides hydroxycarboniques en C₂ à C₇ monovalents ou polyvalents, les produits d'addition de l'oxyde d'éthylène et/ou de l'oxyde de propylène à des alcanols en C₃ à C₂₀ monovalents ou polyvalents, les huiles de paraffine liquides, les huiles isoparaffiniques, en particulier l'isodécane, l'isoundécane, l'isododécane, l'isotridécane, l'isotétradécane, l'isopentadécane, l'isohexadécane et l'isoicosane, les hydrocarbures synthétiques, tels que le polyisobutène ou les polydécènes, et les hydrocarbures alicyclique, les alcools gras ramifiés saturés ou insaturés ayant de 6 à 30 atomes de carbone, les mélanges d'alcools de Guerbet et d'esters d'alcools de Guerbet, les esters symétriques, asymétriques ou cycliques de l'acide carbonique avec les alcools gras, les triglycérides d'acides gras en C₈ à C₃₀ linéaires ou ramifiés, saturés ou insaturés, éventuellement hydroxylés, les esters d'acides dicarboxyliques d'alcanols en C₂ à C₁₀ linéaires ou ramifiés, les di-n-alkyléthers ayant un total de 12 à 36, en particulier 12 à 24 atomes de carbone, et des mélanges des huiles susmentionnées.

8. Composition selon l'une des revendications précédentes, **caractérisée en ce que** l'au moins une huile de support b), liquide dans des conditions normales, comporte au moins une huile isoparaffinique, choisie en particulier parmi l'isodécane, l'isoundécane, l'isododécane, l'isotridécane, l'isotétradécane, l'isopentadécane, l'isohexadécane et l'isoicosane.

9. Composition selon l'une des revendications 1, 2, 3, 4, 5, 6, 7 ou 8, **caractérisée en ce qu'**elle contient 0 à 1% en poids maximum de cyclométhicone.

10. Composition selon l'une des revendications 1, 2, 3, 4, 5, 6, 7, 8 ou 9, **caractérisée en ce que** l'huile de support b), liquide dans des conditions normales, est constituée d'un mélange b)i) d'une huile ester choisie parmi le palmitate de 2-éthylhexyle, le myristate d'isopropyle et le palmitate d'isopropyle, et b)ii) d'au moins une huile isoparaffinique choisie parmi l'isodécane, l'isoundécane, l'isododécane, isotridécane, isotétradécane, l'isopentadécane, l'isohexadécane et l'isoicosane, et b)iii) de 0 à 1% en poids maximum de cyclométhicone.

11. Procédé cosmétique non thérapeutique destiné à réduire et/ou réguler la transpiration et/ou l'odeur corporelle, dans lequel une composition de l'invention ou préférée selon l'invention selon l'une des revendications 1, 2, 3, 4, 5, 6, 7, 8, 9, ou 10 est appliquée dans une quantité efficace sur la peau, de préférence sur la peau dans la région des aisselles.

12. Utilisation cosmétique non-thérapeutique d'une composition anti-transpirante selon la revendication 1, 2, 3, 4, 5, 6, 7, 8, 9 ou 10 pour diminuer et/ou réguler la transpiration et/ou l'odeur corporelle.

13. Utilisation d'au moins un polyéther alkylé de la formule générale ACTIVATEUR-(I) 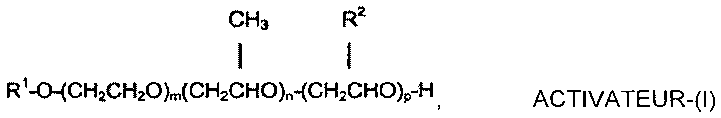 dans laquelle
R¹ est un radical hydrocarboné aliphatique ayant 1 à 3 atomes de carbone,
R² est un radical hydrocarboné aliphatique ayant 8 à 30 atomes de carbone,
m est un nombre rationnel compris entre 10 et 50,
n est un nombre rationnel compris entre 0 et 10,
p est un nombre rationnel compris 1 entre 10 dans une composition anti-transpirante contenant au moins un substance active anti-transpirante et 0 à 7% en poids, de préférence 0 à 3% en poids, d'eau libre, pour améliorer la diminution de la transpiration, **caractérisée en ce que** le polyéther alkylé de la formule générale ACTIVATEUR-(I) est présent dans une composition selon l'une quelconque des revendications 1 à 10.
